(19) Europäisches Patentamt / European Patent Office / Office européen des brevets

(11) **EP 3 981 402 A1**

(12) **EUROPEAN PATENT APPLICATION**
published in accordance with Art. 153(4) EPC


(43) Date of publication:
**13.04.2022 Bulletin 2022/15**

(21) Application number: **21745202.8**

(22) Date of filing: **01.06.2021**

(51) International Patent Classification (IPC):
***A61K 31/454*** (2006.01) ***A61K 31/444*** (2006.01)
***A61K 9/00*** (2006.01) ***A61P 31/10*** (2006.01)

(52) Cooperative Patent Classification (CPC):
**A61K 9/00; A61K 31/444; A61K 31/454; A61P 31/10**

(86) International application number:
**PCT/KR2021/006785**

(87) International publication number:
**WO 2022/035031 (17.02.2022 Gazette 2022/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.08.2020 KR 20200102603**
**09.02.2021 PCT/KR2021/001702**



(71) Applicant: **BL&H Co., Ltd.**
**Seoul 08594 (KR)**

(72) Inventor: **The designation of the inventor has not yet been filed**

(74) Representative: **Office Freylinger**
**P.O. Box 48**
**8001 Strassen (LU)**


(54) **PHARMACEUTICAL COMPOSITION FOR PREVENTION OR TREATMENT OF TINEA**


(57) The composition of the present invention has a wide range of antimicrobial effects including antifungal against Trichophyton rubrum, Trichophyton mentagrophytes, Aspergillus niger, Candida albicans, Epidermophyton floccosum and Microsporum canis, which cause Tinea, and antibacterial effects against Pseudomonas aeruginosa, causative bacteria of green nail syndrome, and has excellent drug permeability to the skin and nail, so it can be used as a therapeutic agent for Tinea unguium, Tinea corporis and green nail syndrome.

In addition, since the pharmaceutical composition of the present invention does not undergo changes in physical properties even when stored at 65°C for 5 weeks or more, the stability of the composition can be ensured.


FIG. 1

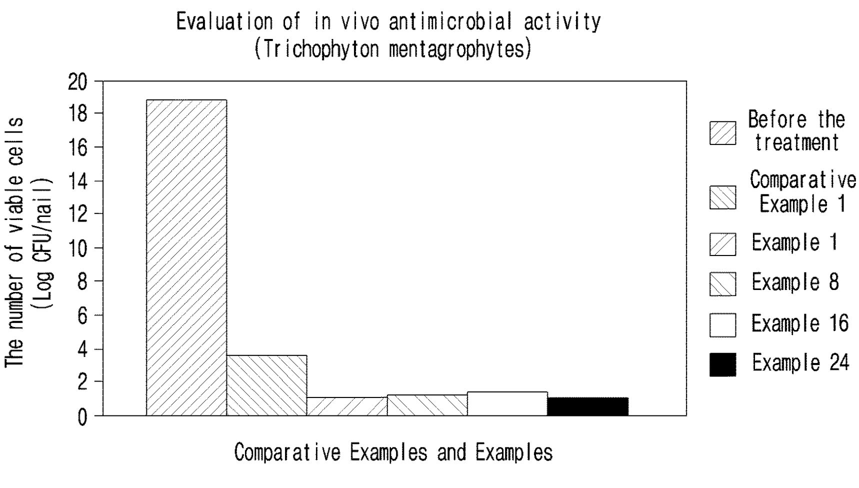





Printed by Jouve, 75001 PARIS (FR)

## Description

## BACKGROUND OF THE INVENTION

### 1. Field of the Invention

The present invention relates to a pharmaceutical composition for preventing or treating Tinea unguium, Tinea corporis and green nail syndrome, comprising efinaconazole and octenidine as active ingredients.

### 2. Description of the Related Art

Tinea is a general term for skin diseases caused by dermatophytes parasitizing the stratum corneum of the skin, hair, and nails while living and multiplying with a protein called keratin, a major component of the stratum corneum, as a nutrient. Tinea is also called dermatophytosis, superficial mycosis or athlete's foot.

Tinea is classified into Tinea captis, Tinea corporis, Tinea cruris, Tinea barbae, Tinea faciel, Tinea manus, Tinea pedis, and Tinea unguium according to the site of occurrence [Byung Ho Oh, Kyu Joong Ahan. "Drug Therapy of Dermatophytosis" J Korean Med Assoc. 2009, 52(11):1109-1114].

According to a press release from the Ministry of Food and Drug Safety Korea in 2017, among domestic Tinea patients, Tinea unguium was the most common, accounting for 46.9% of all Tinea patients, followed by Tinea pedis 30.8%, Tinea corporis 12.9%, Tinea manus 1.4%, and others 8.0%. Tinea unguium and Tinea pedis accounted for about 80% of all Tinea patients (Ministry of Food and Drug Safety Press Release, 2017.7.5.).

Tinea is transmitted through hot and humid climate, work environment, sweat, malnutrition, and contact with an infected person. In a place where people gather, such as a bathroom or swimming pool, it is transmitted to other parts of oneself or family members through skin peels, toenails, or debris from a Tinea patient, or by contact through towels or indoor shoes. In some cases, Tinea can also be infected as the skin is damaged by mechanical stimulation due to moderate humidity and walking.

The causative bacteria of Tinea may vary depending on the patient and the site of occurrence, but in general, there are Trichophyton rubrum, Trichophyton mentagrophytes, Epidermophyton floccosum, Candida albicans as yeast, Aspergillus niger as mold, and Rarely, Microsporum canis. Among them, Trichophyton rubrum accounts for 60% and Trichophyton mentagrophytes account for 20%. In particular, as the causative bacteria of Tinea unguium, Trichophyton rubrum accounts for 91.0% and Trichophyton mentagrophytes account for 7.7% [Uwe Wollina, Holger Haenssle, Dtsch Arztebl Int 2016;113(29-30):509-518].

Tinea unguium is Tinea that occurs on the nails or toenails, also called onychomycosis or nail fungus. Tinea unguium accounts for about half of all Tinea patients, and the recurrence and re-infection rates are 20-25%, and the risk of transmission within the patient's family is high as 44-47%. The typical symptoms of Tinea unguium are thickening, whitening, and brittleness of the nails or toenails. In particular, when Tinea unguium progresses for a long time, onycholysis, the gradual separation of the nails from the skin, may occur. When the symptoms become severe, permanent damage and deformation of the nails can cause cosmetic and functional impairment, which affects the quality of life [Yang Won Lee. "Treatment of onychomycosis" J Korean Med Assoc. 2019 July; 62(7):385-391].

Nails that protect fingers and toes can be mainly divided into four parts: nail plate, nail bed, nail fold, and nail matrix. The lower part of the distal nail part is hyponychium, and the part connected to the nail plate in the proximal nail fold is epionychium (cuticle). Nails are up to 100 times thicker than the stratum corneum of the skin. Hard alpha-keratin, a major component of the nail, is a fibrous structural protein characterized by a high content of cysteine that readily forms heat-resistant crosslinks. In general, the nails are very resistant to penetration of molecules such as drug substances.

Since drug absorption is poor due to the thick and dense nail plate, it is important to increase the permeability of the drug penetrating into the dense keratinous nail plate for the effective treatment of Tinea unguium.

Tinea unguium is classified according to the route and location where the fungus penetrates the nail and has different characteristics. According to the disease information of the Association of Korean Medical Clinical Dermatology, it is classified into five types as follows (Association of Korean Medical Clinical Dermatology; Disease Information, 2017.12.18).

①DLSO (Distal and Lateral Subungal Onychomycosis): It is a common symptom found in 15-20% of the population in their 40s ~ 60s. It mainly occurs on the toenails, but if athlete's foot is not present, it can also occur on the nails. The causative bacterium is T. rubrum. The causative bacteria penetrate from the distal part to the proximal part of the nail, making the bottom of the nail plate cloudy, and causing onycholysis as the stratum corneum thickens. Eventually, the nail plate is destroyed and a yellowish-brown abnormal pigment is often observed. In Korea, the toenail fungus type predominates, and the DLSO type accounts for 81.7%.

②PSO (Proximal Subungl Onychomycosis): This symptom occurs when infection occurs through the lower

side of the proximal nail fold. Infection occurs through the proximal nail fold, stratum corneum, and cuticle. When infected, white spots appear from the proximal cuticle of the nail and gradually spread to the distal part, and the shape of the nail is relatively normal. This symptom is not common and tends to occur frequently in immunodeficient patients such as HIV patients.

③WSO (White Superficial Onychomycosis): This symptom occurs when a fungal infection occurs through the dorsal side of the nail and is usually limited to the toenail. When infected, small white plates with clear boundaries appear on the dorsal side of the nail plate, and as they grow larger, the plates merge and gradually invade the entire nail plate. The surface of the affected area becomes white and rough, and becomes softer than the normal nail. Since the destruction of the nail plate is rare, patients often live without recognizing the lesion. In the case of penetrating only the nail plate, a characteristic "white island" phenomenon is shown in which a small white island-like part appears on the nail.

④EO (Endonyx Onychomycosis): It is the most recently known type of fungal infection of the nail, and it invades the nail through the medullary like DLSO (distal and lateral subungal onychomycosis), but does not cause onycholysis or thickening. Milky white spots appear by invading the keratin of the nail, and bacteria infected with this form can cause Tinea captis.

⑤TDO (Total Dystrophic Onychomycosis): If the above four types of lesions continue to progress, secondary TDO (total dystrophic Onychomycosis) is induced. That is, when TDO occurs, the nail is broken, lost and thickened, and an abnormal nail shape filled with nail keratin debris appears. Primary TDO occurs frequently in patients with immune depression and chronic mucocutaneous candidiasis. When TDO occurs, the nails gradually thicken and turn yellowish-brown, and Candida bacteria invade the skin around the nail and secondary keratinize the surrounding skin. The type that DLSO, PSO, and WSO continue to reach at the end is TDO. As onychomycosis progresses, the entire nail is destroyed, and if this symptom is left unattended for a long time, it becomes TDO.

Tinea corporis is Tinea that occurs in the skin area other than the nail, and includes Tinea pedis, Tinea manus, Tinea corporis, Tinea captis, and Tinea cruris.

Tinea manus and Tinea pedis refer to Tinea with symptoms in the hands and feet, respectively. These symptoms can be divided into the following three categories: interdigital type with peeling between the toes, oozing and itching; hyperkeratoic type, in which the skin of the palms and soles becomes thick and the keratin fall into a fine powder when scratched; and vesicular type with itching and large and small blisters on the palms and toes. If Tinea manus and Tinea pedis are left untreated, secondary infection of the nails and toenails may be induced and Tinea unguium may occur.

Tinea capitis is Tinea on the head and is mainly transmitted through contact with pets such as dogs and cats. When Tinea capitis occurs, gray or red scaly lesions appear on the scalp, and hair loss may occur in some places. In severe cases of inflammation, it can lead to extensive hair loss, and pus pockets or enlarged lymph nodes may appear.

Tinea cruris is Tinea that appears in the inguinal region where the skin overlaps, and mainly occurs in adult males. Tinea cruris is characterized by the appearance of a lesion with a well-defined round or semicircular reddish-brown scale. In severe cases, it can spread to the genitals, around the anus, buttocks, and the inside of the thighs, and scales and pigmentation may remain.

Tinea corporis is Tinea that occurs on the skin of the body except for the head, hands, feet, and groin. This symptom is mainly caused by fungal transfer from the patient's own Tinea unguium, and can also be caused by poor humidity control in the living space or by holding a pet. This symptom shows a spreading form of round-shaped lesions of various sizes with clear borders, and may be accompanied by spots covered with debris or inflammation.

GNS (Green Nail Syndrome) is one of the diseases associated with Tinea unguium. It is a disease that causes a green discoloration of the nail plate (green-yellow, green-brown or green-black) by producing green pigments such as Pyocyanin, Pyoverdin and Pyorubin as Pseudomonas aeruginosa penetrates and reproduces in the abnormally lifted nail.

Other diseases associated with Tinea unguium include Paronychia, in which inflammation is caused by Candida albicans (yeast) and Staphylococcus aureus (Gram-positive bacterium); Onycholysis, in which the nails are separated from the skin; Onychotosis, in which part or all of the nails are periodically shed; and Onychodystrophy, in which the nail surface becomes bumpy or discolored.

Antifungal agents used in the treatment of Tinea include polyenes, azoles, allylamines, benzylamines, morpholines, hydroxypyridones and oxaboroles.

As antifungal agents for topical treatment that are currently commercialized and distributed, polyenes include amphotericin B (0.1% cream). Azoles are divided into imidazoles and triazoles. Imidazoles include clitrimazole (1% cream/lotion), ketoconazole (2% cream/gel), econazole (1% cream), miconazole (2% cream/gel), biponazole (1% cream), oxyconazole (1% cream/lotion), thioconazole (1% cream), sertaconazole (2% cream), luconazole (1% cream), everconazole (1%) cream), sulconazole (1% cream), penticonazole (2% cream), etc. Triazoles include fluconazole (0.5% gel) and efinaconazole (10% liquid). Vinafine (1% cream/spray) and naftifene (1% cream). Allylamines include terbinafine (1% cream/spray) and naftifene (1% cream). Benzylamines include butenafine (1% cream), morpholines include amorolphine (5% nail lacquer), hydroxypyridones include ciclopirox (8% nail lacquer), and oxaboroles include tababorol (5% liquid) (Shital Amin, Poojary. "Topical Antifungals; A Review and their Role Management of Dermatophytoses" Clinical Dermatology Review 2017;1;s24-s29.).

Among them, amorolphine (5% nail lacquer), ciclopirox (8% nail lacquer), tababolol (5% liquid), and efinaconazole (10% liquid) have been marketed for a long time as a treatment for Tinea unguium.

In particular, JUBULIA (FDA approved in 2014; Kaken Pharmaveutical Co, Ltd), a product containing 10% efinaconazole, has an activity of inhibiting lanosterol 14 $\alpha$-demethylase in the biosynthesis pathway of ergosterol, an essential element for fungal cell viability by maintaining the membrane fluidity and permeability barrier of the fungal cell membrane, and is widely sold worldwide as a treatment for Tinea unguium.

Since efinaconazole is known to be more than twice as effective as the existing topical agents, the demand as a therapeutic agent for Tinea unguium is increasing. Accordingly, studies on increasing the stability and permeability of efinaconazole and methods of increasing the yield during manufacturing are continuing.

The prior art for efinaconazole includes the following. Korean Patent No. 10-2181155, "method for preparation of efinaconazole in ionic liquid medium" discloses a preparation method that can easily obtain the final compound efinaconazole with high purity and high yield by reducing the related substances and shortening the reaction time compared to the conventional methods by using an ionic liquid instead of an organic solvent when manufacturing efinaconazole. Korean Patent Publication No. 10-2020-0140681, "pharmaceutical compositions for topical administration in the form of solution comprising efinaconazole" discloses an efinaconazole solution that significantly increases the skin and nail penetration rate and permeability of efinaconazole comprising diethylene glycol monoethyl ether; or a combination of diethylene glycol monoethyl ether and isopropyl myristate as a non-volatile solvent. In addition, Korean Patent Publication No. 10-2016-0068812, "stabilized efinaconazole compositions" discloses efinaconazole compositions with increased stability containing efinaconazole, butylated hydroxytoluene, a salt of ethylenediaminetetraacetic acid, and citric acid.

Octenidine is a widely used antiseptic and disinfectant, and is used not only for disinfection of wounds, but also for antibacterial and antiviral purposes. Octenidine binds to bacterial phospholipids and damages cell membranes to kill cells, has no systemic toxicity and allergic reactions, and has few side effects, so it is a disinfectant that can be used for pregnant women, lactating women, and newborn babies.

The prior art for octenidine includes the following. Korean Patent Publication No. 10-2018-0046644, "compositions for sterilization and disinfection comprising octenidine and benzalconium chloride" discloses compositions for sterilization and disinfection that can effectively sterilize pathogenic microorganisms such as Pseudomonas aeruginosa, Vibrio cholerae, food poisoning bacteria, Escherichia coli, and Klebsiella pneumoniae at a low concentration by adding benzalkonium chloride, a type of ammonium salt preparation, to octenidine.

On the other hand, in the liquid form of a therapeutic agent for Tinea unguium, a volatile solvent is added to reduce stickiness by drying the solution within a short time after application. Cyclomethicone is one of the components of silicone oil that makes the spreadability better. Since cyclomethicone has volatility, it is added to nail lacquer to reduce stickiness when used in cosmetic products and liquid form of Tinea unguium treatment.

However, the European Chemicals Agency (ECHA) has assessed cyclomethicone as a persistent/bioaccumulative/toxic (PBT) and highly persistent/highly bioaccumulative (vPvB) substance, and it is classified into Repr.2 (substance suspected of reproductive toxicity) and Aquatic Chronic 4 (may cause long-term harmful effects on aquatic organisms) by the Regulations on Classification, Labeling and Packaging of Chemicals (CLP regulation). Therefore, in recent years, considering the harmfulness of cyclomethicone, some countries have restricted its use.

While conducting studies on the treatments for Tinea unguium, Tinea corporis and green nail syndrome, the present inventors confirmed that the therapeutic effect on Tinea unguium and Tinea corporis was increased and the antibacterial effect on Pseudomonas aeruginosa, the causative bacterium of green nail syndrome, was increased when the efinaconazole and octenidine combination formulation was administered compared to when efinaconazole or octenidine was administered alone. In addition, the present inventors have completed the present invention by confirming that the drug permeability and drug penetration rate were increased, and the stability was maintained without any change in physical properties even when stored for a long time at high temperature.

## SUMMARY OF THE INVENTION

It is an object of the present invention to provide a pharmaceutical composition for preventing or treating Tinea unguium, Tinea corporis and green nail syndrome, comprising efinaconazole and octenidine as active ingredients.

It is another object of the present invention to provide a use for the prevention or treatment of Tinea unguium, Tinea corporis and green nail syndrome.

It is another object of the present invention to provide a method for preventing or treating Tinea unguium, Tinea corporis and green nail syndrome by applying the pharmaceutical composition to an affected area.

To achieve the above objects, the present invention provides a pharmaceutical composition for preventing or treating Tinea unguium, Tinea corporis and green nail syndrome, comprising efinaconazole or a pharmaceutically acceptable salt thereof; and octenidine or a pharmaceutically acceptable salt thereof as active ingredients.

The pharmaceutical composition further includes an additive; and the additive can be at least one selected

from the group consisting of solvents, antioxidants, chelating agents, penetrants, film-formers, disintegrants, binders, lubricants, coating agents, emulsifiers, pressure sensitive adhesives, surfactants, gelling agents, solubilizers, buffering agents, wetting agents, osmotic pressure regulators, aerosol propellants, sweetening agents, coloring agents, flavoring agents, stabilizing agents, bases, pH modifiers and vehicles.

The content of efinaconazole or the pharmaceutically acceptable salt thereof can be 0.001 ~ 20 weight% based on the total weight of the composition.

The pharmaceutically acceptable salt of efinaconazole can be at least one selected from the group consisting of inorganic ionic salts, inorganic acid salts, organic acid salts, sulfonic acid salts, amino acid salts and amine salts.

The content of octenidine or the pharmaceutically acceptable salt thereof can be 0.001 ~ 10 weight% based on the total weight of the composition.

The pharmaceutically acceptable salt of octenidine can be at least one selected from the group consisting of inorganic ionic salts, inorganic acid salts, organic acid salts, sulfonic acid salts, amino acid salts and amine salts.

The pH of the pharmaceutical composition can be 2 - 10.

The surface tension of the pharmaceutical composition can be 1 ~ 75 dyn/cm.

The water content of the pharmaceutical composition measured by Karl Fischer's method can be 0.001 ~ 95%.

The daily dosage of the pharmaceutical composition can be 0.0001 ~ 300 g/day.

The pharmaceutical composition can have a dosage form selected from the group consisting of solutions, ointments, lotions, creams, gels, emulsions, suspensions, powder compacts, pastes, sticks, plasters, patches, tablets, powders, granules, capsules, gums, troches, mucoadhesives, inhalants, microcapsules, detergents, baths, injections, suppositories, liposomes and sprays.

The pharmaceutical composition can be formulated in the form of inhalation or oral preparations, parenteral preparations including external preparations, and injections.

The pharmaceutical composition is colorless or pale yellow, and can maintain the same color even after storage at 65°C for at least 5 weeks.

The density of the pharmaceutical composition can be 0.001 ~ 2 g/cm$^3$.

## ADVANTAGEOUS EFFECT

The composition of the present invention has an advantage in that it has excellent therapeutic effects on Tinea (for example, Tinea unguium and Tinea corporis), and can also treat green nail syndrome.

According to various embodiments of the present invention, the composition of the present invention has a much higher antifungal effect than the conventional efinaconazole single-component preparation, and has an excellent antibacterial effect against gram-negative bacteria (eg, Pseudomonas aeruginosa) and gram-positive bacteria (eg, Staphylococcus aureus). In addition, the composition of the present invention has high drug compliance due to its rapid antibiotic effect, and has a fast drug penetration rate into the skin or nail and excellent drug permeability.

In addition, according to various embodiments of the present invention, since the pharmaceutical composition of the present invention does not undergo changes in physical properties (eg, occurrence of precipitation or discoloration, changes in the content of active ingredients, etc.) even when stored at 65°C for 5 weeks or more, the stability of the composition can be ensured.

That is, the composition of the present invention has a better therapeutic effect on Tinea by increasing drug permeability compared to the conventional efinaconazole single-component preparation, and as the thermal stability is improved compared to the conventional efinaconazole single-component preparation, it can be stored stably for a long time.

## BRIEF DESCRIPTION OF THE DRAWINGS

Figure 1 is a graph showing the results of *in vivo* antifungal experiments according to Comparative Example 1 and various Examples.

Figure 2 is a graph showing the *in vivo* antibacterial effects according to Comparative Example 1 and various Examples.

Figure 3 is a graph showing the cumulative permeated concentration of efinaconazole for 10 daysaccording to Comparative Example 1 and various Examples.

Figure 4 is a graph showing the results of *in vivo* antifungal experiments according to Example 1 and Comparative Example 2.

Figure 5 is a graph showing the *in vivo* antibacterial effects according to Example 1 and Comparative Example 2.

Figure 6 is a graph showing the cumulative permeated concentration of octenidine dihydrochloride for 10 days

according to Example 1 and Comparative Example 2.

## DESCRIPTION OF THE PREFERRED EMBODIMENTS

In this specification, it should be noted that references to "one" or "a" of the present invention are not necessarily to the same embodiment, and they mean at least one.

In the following embodiments, terms such as 1st, 2nd, etc. are used for the purpose of distinguishing one component from another, not in a limiting sense.

In the following embodiments, the singular expression includes the plural expression unless the context clearly dictates otherwise.

In the following embodiments, terms such as include or have means that the features or components described in the specification are present, and the possibility that one or more other features or components may be added is not excluded in advance.

Hereinafter, the present invention is described in detail.

The present invention provides a pharmaceutical composition for preventing or treating Tinea unguium, comprising efinaconazole or a pharmaceutically acceptable salt thereof; and octenidine or a pharmaceutically acceptable salt thereof as active ingredients.

Tinea unguium is a disease caused by the invasion of fungus into the nail, that is, the finger nails and toenails. The typical symptoms are thick, white, and brittle nails.

Tinea unguium can be induced by at least one selected from the group consisting of Trichophyton rubrum, Trichophyton mentagrophytes, Aspergillus niger, Candida albicans, Epidermophyt floccosum and Microsporum canis.

In clinical practice, Trichophyton rubrum is detected as the causative organism in about 90% of Tinea unguium, and Trichophyton mentagrophytes and Trichophyton epidermis are also isolated. Candida is often isolated from the finger nails.

The efinaconazole (CAS registration number: 164650-44-6) of the present invention is a triazole-based antifungal agent having three nitrogen groups and exhibits activity against a wide range of pathogenic fungi. The chemical name of efinaconazole is (2R,3R)-2-(2,4-difluorophenyl)-3-(4-methylenepiperidin-1-yl)-1-(1H-1,2,4-triazol-1-yl)butan-2-ol. The structural formula of efinaconazole is represented by the following formula 1.

[Formula 1]

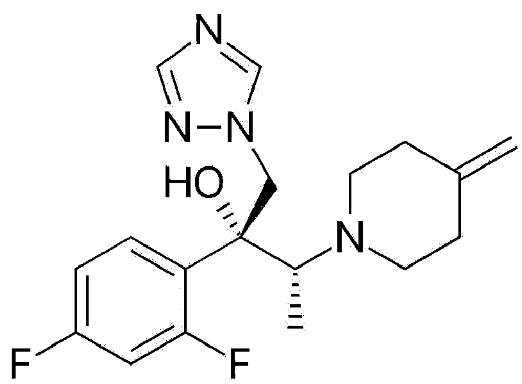

The molecular formula of efinaconazole is $C_{18}H_{22}F_2N_4O$ (molecular weight: 348.39, melting point: 192~195°C), and efinaconazole is a white or pale yellow powder.

Efinaconazole has antibiotic effects on Trichophyton rubrum, Trichophyton mentagrophytes, Aspergillus niger, Candida albicans, Epidermophyt floccosum and Microsporum canis.

Efinaconazole has an activity of inhibiting lanosterol 14 $\alpha$-demethylase in the biosynthesis pathway of ergosterol, an essential element for fungal cell viability. Since efinaconazole exhibits high reversibility compared to other drugs, it is effective in treating fungal infections of the nail.

The pharmaceutically acceptable salt of efinaconazole can be at least one selected from the group consisting of inorganic ionic salts, inorganic acid salts, organic acid salts, sulfonic acid salts, amino acid salts and amine salts.

In this specification, the term "salt" refers to an acid addition salt formed by a pharmaceutically acceptable free acid. Accordingly, the pharmaceutically acceptable salt means a salt commonly used in the pharmaceutical industry, for example, inorganic ion salts prepared from calcium, potassium, sodium or magnesium; inorganic acid salts prepared from hydrochloric acid, nitric acid, phosphoric acid, hydrobromic acid, iodic acid, perchloric acid or sulfuric acid; organic

acid salts prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid or vanillic acid; sulfonic acid salts prepared from methanesulfonic acid, ethanesulfonic acid, benzenesulfonic acid, salicylic acid, p-toluenesulfonic acid or naphthalenesulfonic acid; amino acid salts prepared from glycine, arginine or lysine; or amine salts prepared from trimethylamine, triethylamine, ammonia, pyridine or picoline; but the types of salts in the present specification are not limited by these salts listed above.

The content of efinaconazole or the pharmaceutically acceptable salt thereof can be 0.001 ~ 20 weight% based on the total weight of the composition.

The content range of efinaconazole or the pharmaceutically acceptable salt thereof can be 0.001 ~ 20 weight%, 0.01 ~ 20 weight%, 0.05 ~ 19 weight%, 0.1 ~ 18 weight%, 0.5 ~ 17 weight%, 1 ~ 16 weight%, 2 ~ 15 weight%, 3 ~ 14 weight%, 4 ~ 13 weight%, 5 ~ 20 weight%, 8 ~ 20 weight%, 10 ~ 16 weight%, 1 ~ 10 weight%, 5 ~15 weight%, or 10 ~ 20 weight%.

The content of efinaconazole or the pharmaceutically acceptable salt thereof can be applied in an amount effective for preventing or treating Tinea unguium. The level of an amount effective for preventing or treating Tinea unguium can be determined according to the type of disease, severity, drug activity, susceptibility to drug, administration time, administration route, excretion rate, duration of treatment, factors including drugs used concurrently and other factors well known in the medical field.

In consideration of all of the above factors, it is important to administer an amount that can obtain the maximum effect with a minimum amount without side effects, which can be easily determined by those skilled in the art.

The octenidine (CAS registration number: 71251-02-0) of the present invention is represented by the following formula 2. The molecular formula of octenidine is $C_{36}H_{62}N_4$, and the molecular weight is 550.9. Since octenidine has antibacterial activity, it is effective in sterilizing bacteria.

[Formula 2]

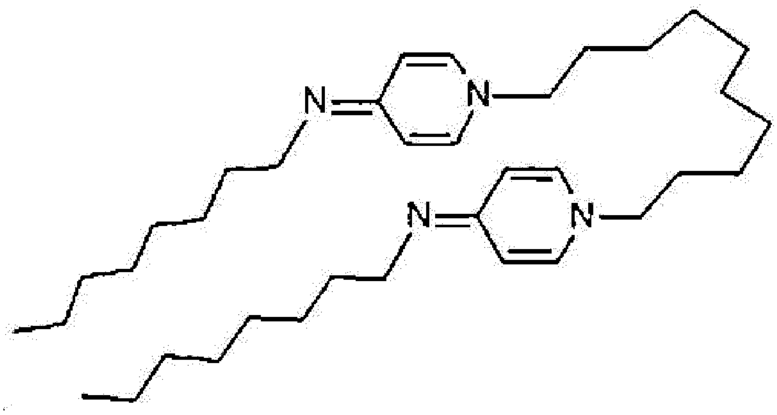

The pharmaceutically acceptable salt of octenidine can be at least one selected from the group consisting of inorganic ionic salts, inorganic acid salts, organic acid salts, sulfonic acid salts, amino acid salts and amine salts. For example, the pharmaceutically acceptable salt of octenidine can include inorganic ion salts prepared from calcium, potassium, sodium or magnesium; inorganic acid salts prepared from hydrochloric acid, nitric acid, phosphoric acid, hydrobromic acid, iodic acid, perchloric acid or sulfuric acid; organic acid salts prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid or vanillic acid; sulfonic acid salts prepared from methanesulfonic acid, ethanesulfonic acid, benzenesulfonic acid, salicylic acid, p-toluenesulfonic acid or naphthalenesulfonic acid; amino acid salts prepared from glycine, arginine or lysine; or amine salts prepared from trimethylamine, triethylamine, ammonia, pyridine or picoline; but the types of octenidine salts are not limited by these salts listed above.

In the present invention, the pharmaceutically acceptable salt of octenidine can be applied as octenidine dihydrochloride (CAS registration No. 70775-75-6). Octenidine dihydrochloride is a white or pale yellow powder, and is a pyridine-based compound.

The chemical name for octenidine dihydrochloride is N-octyl-1-[10-(4-octyliminopyridin-1-yl)decyl]pyridin-4-imine dihydrochloride. The molecular formula of octenidine dihydrochloride is $C_{36}H_{64}Cl_2N_4$ (molecular weight: 623.84, melting point: 215 ~ 217°C). The structural formula of octenidine dihydrochloride is represented by the following formula 3.

[Formula 3]

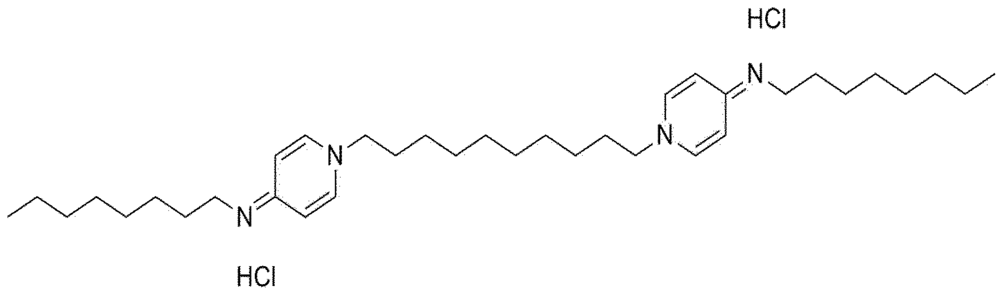

HCl

HCl

Octenidine dihydrochloride binds to phospholipids and anionic polysaccharides in the cell membrane by strongly adsorbing to the cell surface of bacteria that exhibit anions, and decreases cellular functions, causing leakage of plasma membrane and inhibiting mitochondrial functions, thereby exhibiting excellent sterilization effect.
The content of octenidine or the pharmaceutically acceptable salt thereof can be 0.001 ~ 10 weight% based on the total weight of the composition.
The content range of octenidine or the pharmaceutically acceptable salt thereof can be 0.001 ~ 10 weight%, 0.01 ~ 10 weight%, 0.05 ~ 9 weight%, 0.1 ~ 8 weight%, 0.5 ~ 7 weight%, 1 ~ 6 weight%, 2 ~ 5 weight%, or 3 ~ 4 weight%.
The content of octenidine or the pharmaceutically acceptable salt thereof is not limited to the above-described ranges, and can be applied in an amount effective for preventing or treating Tinea unguium.
Since the pharmaceutical composition for preventing or treating Tinea unguium of the present invention can sterilize fungi and bacteria strongly by a complex antibacterial action, it exhibits a very excellent antibiotic effect compared to each single agent.
The pharmaceutical composition for preventing or treating Tinea unguium of the present invention can further include an additive.
The additive can be at least one selected from the group consisting of solvents, antioxidants, chelating agents, penetrants, film-formers, disintegrants, binders, lubricants, coating agents, emulsifiers, pressure sensitive adhesives, surfactants, gelling agents, solubilizers, buffering agents, wetting agents, osmotic pressure regulators, aerosol propellants, sweetening agents, coloring agents, flavoring agents, stabilizing agents, bases, pH modifiers and vehicles.
In this specification, the term "disintegrant" means a component necessary for a solid to expand in order to be decomposed in the digestive tract before it is absorbed.
In this specification, the term "binder" means a substance that imparts uniform adhesion, solidification and/or consistency to a mixture.
In this specification, the term "lubricant" means a substance used to reduce adhesion to a tableting punch by providing slipperiness during the preparation of tablets.
In this specification, the term "emulsifier" means a substance for preventing separation of immiscible liquid substances.
In this specification, the term " pressure sensitive adhesive " means a substance that adheres well to each other with other materials and is then detachable.
In this specification, the term "solubilizer" means a substance that increases the solubility of a substance.
In this specification, the term "buffering agent" means a substance added to control the hydrogen ion concentrationexponent.
In this specification, the term "sweetening agent" means a substance that gives a sweet taste.
In this specification, the term "flavoring agent" means a substance added to pharmaceuticals and/or foods to add a particular flavor.
In this specification, the term "stabilizing agent" means a substance added to prevent decomposition by acid, moisture, light, and the like.
In this specification, the term "base" means an inactive ingredient that can be used as a vehicle for an active medicinal product.
In this specification, the term "pH modifier" means a substance that changes the acid-alkali balance.
The solvent can be at least one organic solvent selected from the group consisting of alkane, alkene, alcohol, ester, ketone, and ether solvents. For example, the solvent can include ethanol, isopropyl alcohol, phenoxy ethanol, acetone, butyl acetate, and ethyl acetate. Preferably, ethanol, isopropyl alcohol, acetone, etc. can be used as the solvent.
The antioxidant can be at least one selected from the group consisting of BHT (butylhydroxytoluene), BHA (butylhydroxyanisole), ascorbic acid, tocopherol, caffeic acid, ferulic acid, propyl gallate, cysteine, N-acetylcystein, sodium sulfite and mixtures thereof. Preferably, butylhydroxytoluene, butylhydroxyanisole, ascorbic acid, tocopherol, caffeic

acid, ferulic acid, etc. can be used as the antioxidant.

The chelating agent is a sequestering agent that binds to oxygen, nitrogen, or various metal ions to inactivate them. The chelating agent can be at least one selected from the group consisting of glutamic acid, methylglycine, sodium pyrophosphate, disodium EDTA, tetrasodium EDTA and mixtures thereof. Preferably, glutamic acid, sodium pyrophosphate, disodium EDTA, tetrasodium EDTA, etc. can be used as the chelating agent.

The penetrant is a substance added to enhance the therapeutic effect by increasing the penetration of the drug into the nail or skin. The penetrant can be at least one selected from the group consisting of diethylhexyl adipate, disopropyl sebacate, diethyl sebacate, diethylhexyl succinate, oleyl oleate, sodium laurylsulfate, carpric/carprylic triglyceride, thioglycolic acid, mercaptoethanol, 1,2-hexanediol, dipropylene glycol, 2-methyl-1,3-propanediol, propylene glycol and mixtures thereof. Preferably, 1,2-hexanediol, dipropylene glycol, methylpropanediol, diethyl sebacate, diethylhexyl succinate, oleyl oleate, capric/caprylic triglyceride, propylene glycol, etc. can be used as the penetrant.

The film-former can be at least one selected from the group consisting of povidone, copovidone, chitosan, methacrylic acid copolymer, polyvinyl alcohol, hydroxyethyl cellulose, sodium alginate and mixtures thereof. Preferably, povidone, copovidone, polyvinyl alcohol, hydroxyethyl cellulose, etc. can be used as the film-former.

The pH modifier can be at least one selected from the group consisting of hydrochloric acid, phosphoric acid, acetic acid, citric acid, succinic acid, lactic acid, malic acid, tartaric acid, alkaline earth metal hydroxide, carbonate, sodium hydroxide, sodium carbonate, ethanolamine, lauryl glycoside, coco glycoside, lysine, arginine, triamineethanol, sodium citrate, calcium citrate, diisopropylamine and mixtures thereof. Preferably, citric acid, tartaric acid, sodium hydroxide or sodium citrate can be used to adjust the pH range of the composition to 2 ~ 10.

The vehicle is an additive that can maintain the shape of the composition. The vehicle can be at least one selected from the group consisting of water for injection (WFI), purified water, physiological saline, glycerin, mineral oil, soybean oil, lanolin oil, sorbitol, carboxylmethyl cellulose, gellatin, hydroxyethyl cellulose, sodium alginate, ethyl cellulose, octylacrylamide/acrylate copolymers and mixtures thereof. Preferably, purified water, glycerin, carboxymethyl cellulose, sodium alginate or sorbitol can be used as the vehicle.

The pharmaceutical composition can further include a plasticizer, a sustained-release agent, a foaming agent, a curing agent, a stabilizer, a light-shielding agent, a masking agent, a viscosity regulator, and the like for improved feeling of use, improved administration in the body, and/or stability of the formulation. As the above-mentioned plasticizer, sustained-release agent, foaming agent, curing agent, stabilizer, light-shielding agent, masking agent and viscosity regulator, the components commonly used in the art can be used, examples of which are listed in the literature (Raymond C. Rowe. Handbook of Pharmaceutical Excipients 7th edition). In addition, the above-mentioned plasticizer, sustained-release agent, foaming agent, curing agent, stabilizer, light-shielding agent, masking agent and viscosity regulator can be prepared by adding them in various compositions according to the intended use and conditions, and can preferably be added in an amount of 0.01 ~ 5 weight%.

In an embodiment of the present invention, the pharmaceutical composition for preventing or treating Tinea unguium can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, 1,2-hexanediol), 1 ~ 20 weight% of a second penetrant (eg, methylpropanediol), 0.01 ~ 3 weight% of a pH modifier (eg, succinic acid), and 1 ~ 95 weight% of a vehicle.

In another embodiment of the present invention, the pharmaceutical composition for preventing or treating Tinea unguium can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, 1,2-hexanediol), 1 ~ 20 weight% of a second penetrant (eg, methylpropanediol), 1 ~ 15 weight% of a film-former (eg, povidone), 0.01 ~ 3 weight% of a pH modifier (eg, succinic acid), and 1 ~ 95 weight% of a vehicle.

In another embodiment of the present invention, the pharmaceutical composition for preventing or treating Tinea unguium can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, dipropylene glycol), 1 ~ 20 weight% of a second penetrant (eg, methylpropanediol), 0.01 ~ 3 weight% of a pH modifier (eg, succinic acid), and 1 ~ 95 weight% of a vehicle.

In another embodiment of the present invention, the pharmaceutical composition for preventing or treating Tinea unguium can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, dipropylene glycol), 1 ~ 20 weight% of a second penetrant (eg, methylpropanediol), 1 ~ 15 weight% of a film-former (eg, povidone), 0.01 ~ 3 weight% of a pH modifier (eg, succinic acid), and 1 ~ 95 weight% of a vehicle.

The pH of the pharmaceutical composition for preventing or treating Tinea unguium can be 2 ~ 10.

The pH range of the composition can be 2 ~ 10, 4 ~ 10, 5 ~ 7, 5 ~ 6, or 5 ~ 10.

The surface tension of the pharmaceutical composition for preventing or treating Tinea unguium can be 1 ~ 75 dyn/cm.

The surface tension range of the composition can be 1 dyn/cm ~ 75 dyn/cm, 5 dyn/cm ~ 60 dyn/cm, 10 dyn/cm ~ 50 dyn/cm, 15 dyn/cm ~ 65 dyn/cm, 20 dyn/cm ~ 60 dyn/cm, 10 dyn/cm ~ 40 dyn/cm, 15 dyn/cm ~ 30 dyn/cm, 25 dyn/cm ~ 55 dyn/cm, 30 dyn/cm ~ 50 dyn/cm, 41 dyn/cm ~ 50 dyn/cm, 42 dyn/cm ~ 50 dyn/cm, 43 dyn/cm ~ 50 dyn/cm, 44 dyn/cm ~ 50 dyn/cm, 45 dyn/cm ~ 50 dyn/cm, 41 dyn/cm ~ 45 dyn/cm, 41 dyn/cm ~ 44 dyn/cm, 41 dyn/cm ~ 43 dyn/cm or 50 dyn/cm ~ 75 dyn/cm.

In this specification, the term "surface tension", generally referred to in units of dyn/cm, means the force required to increase the unit area of the surface of a liquid or increase the unit area of the interface between two liquids or between a liquid and a gas.

The surface tension described herein was measured by Du Nouey Ring Method using a surface tensiometer (Easy Dyne tensiometer model K20) sold by KRUESS.

The water content of the pharmaceutical composition measured by Karl Fischer's method can be 0.001 ~ 95%.

The water content range of the composition can be 0.001 ~ 95%, 0.1 ~ 85%, 0.5 ~ 75%, 1 ~ 70%, 2 ~ 5%, 3 ~ 5%, 5 ~ 85%, 7 ~ 60%, 8 ~ 55%, 9 ~ 50%, 10 ~ 45%, 10 ~ 20%, 10 ~ 15%, 10 ~ 13%, 15 ~ 40% or 20 ~ 35%.

In this specification, the term "water content" refers to the percentage of water contained in the pharmaceutical composition. The water content described in the present specification was measured by Karl Fischer titration using a Metrohm moisture meter (Metrohm 901 KF Titrando).

The water content described herein is calculated by mathematical formula 1 below.

[Mathematical Formula 1]

$$\text{Amount of water (\%)} = V(\text{mL}) \times f(\text{mg/mL}) \times 100 / \text{mass of sample (mg)}$$

(Herein, V is the amount of the water measurement reagent consumed for titration of a sample, and f is the number of mg of water corresponding to 1 mL of the water measurement reagent.)

f in mathematical formula 1 is calculated by mathematical formula 2 below.

[Mathematical Formula 2]

$$f(\text{mg/mL}) = \text{amount of water (mg)} / \text{amount of water measurement reagent consumed for water titration (mL)}$$

The daily dosage of the pharmaceutical composition for preventing or treating Tinea unguium can be 0.0001 ~ 300 g/day.

The daily dosage range of the composition can be 0.0001 ~ 300 g/day, 0.001 ~ 50 g/day, 0.01 ~ 50 g/day, 0.1 ~ 50 g/day, 0.001 ~ 1 g/day, 0.01 ~ 0.5 g/day, 0.05 ~ 0.1 g/day, 1 ~ 50 g/day, 5 ~ 45 g/day, 10 ~ 40 g/day, 15 ~ 35 g/day, 20 ~ 30 g/day, 25 ~ 100 g/day, 70 ~ 150 g/day, 120 ~ 250 g/day, or 200 ~ 300 g/day.

In addition, the daily dosage can be appropriately adjusted in consideration of the case where the pharmaceutical composition for preventing or treating Tinea unguium is applied as inhalation or oral preparations, parenteral preparations including external preparations, or injections.

The pharmaceutical composition for preventing or treating Tinea unguium can be formulated as a dosage form selected from the group consisting of solutions, ointments, lotions, creams, gels, emulsions, suspensions, powder compacts, pastes, sticks, plasters, patches, tablets, powders, granules, capsules, gums, troches, mucoadhesives, inhalants, microcapsules, detergents, baths, injections, suppositories, liposomes and sprays.

In addition, the pharmaceutical composition for preventing or treating Tinea unguium can be formulated in the form of inhalation or oral preparations, parenteral preparations including external preparations, and injections.

In this specification, the term "powder compact" refers to a dosage form prepared by pulverizing the pharmaceutical composition and compressing the powder by applying pressure. When the pharmaceutical composition is pulverized, the conventional spray drying or freeze drying method can be used, but the present invention is not limited thereto and other known pulverizing methods can be used.

In this specification, the term "stick" refers to a dosage form made in the shape of a solid rod, and a term having the same meaning is "bar".

In this specification, the term "plaster" refers to a topical external preparation prepared by spreading or encapsulating a mixture of a main component and a base or an additive in a cloth or plastic film, and adhering thereof to the affected area on the skin surface, so that the active ingredient can reach the local affected area through the skin.

In this specification, the term "patch" refers to an external preparation that is applied to the skin rather than oral administration or injection and continuously exhibits medicinal effects acting on the whole body.

In this specification, the term "capsule" refers to a dosage form prepared by filling a capsule with medicines in the form of liquid, suspension, semisolid, powder, granule or molded drug, or encapsulating pharmaceutical products into a capsule base. In the present specification, capsules include at least one of hard capsules and soft capsules.

In this specification, the term "microcapsule" refers to an aggregate of small particles encapsulating medicinal products inside a thin film of high molecular materials such as gelatin, polyvinyl alcohol, ethyl cellulose, etc.

In this specification, the term "liposome" refers to a dosage form prepared by encapsulating an active ingredient in an aqueous or lipid membrane of a liposome.

The pharmaceutical composition for preventing or treating Tinea unguium is colorless or pale yellow, and can maintain the same color even after storage at 65°C for at least 5 weeks. That is, the pharmaceutical composition can retain the color of the composition when it was first formulated even after storage at 65°C for at least 5 weeks.

The density of the pharmaceutical composition for preventing or treating Tinea unguium can be 0.001 ~ 2 $g/cm^3$.

The density range of the composition can be 0.001 $g/cm^3$ ~ 2 $g/cm^3$, 0.01 $g/cm^3$ ~ 1.5 $g/cm^3$, 0.1 $g/cm^3$ ~ 1 $g/cm^3$, 0.5 $g/cm^3$ ~ 1.5 $g/cm^3$, or 0.7 $g/cm^3$ ~ 1 $g/cm^3$.

In this specification, the term "density" means a value indicating mass per unit volume, and is generally expressed in units of $g/cm^3$.

On the other hand, when the pharmaceutical composition for preventing or treating Tinea unguium is prepared as an external preparation and applied topically, a method of applying, spraying or attaching the composition to the affected site of a patient in an appropriate amount can be used.

The present invention also provides a pharmaceutical composition for preventing or treating Tinea corporis, comprising efinaconazole or a pharmaceutically acceptable salt thereof; and octenidine or a pharmaceutically acceptable salt thereof as active ingredients.

Tinea corporis is a disease caused by fungi infecting the stratum corneum, which are the outermost layers of the skin, and hair. As the symptoms, the skin becomes festered and swollen with moisture, becomes white, cracked, and exfoliated. Unpleasant foot odor and itching can be accompanied when one sweats a lot.

Tinea corporis can be induced by at least one selected from the group consisting of Trichophyton rubrum, Trichophyton mentagrophytes, Aspergillus niger, Candida albicans, Epidermophyt floccosum and Microsporum canis.

Hereinafter, the detailed description of the pharmaceutical composition for preventing or treating Tinea corporis overlaps with the description of the pharmaceutical composition for preventing or treating Tinea unguium, and thus the description thereof is omitted.

The content of efinaconazole or the pharmaceutically acceptable salt thereof and the content of octenidine or the pharmaceutically acceptable salt thereof in the pharmaceutical composition for preventing or treating Tinea corporis can be applied in the same amount as in the pharmaceutical composition for preventing or treating Tinea unguium.

The content of efinaconazole or the pharmaceutically acceptable salt thereof can be 0.001 ~ 20 weight% based on the total weight of the composition.

The content of octenidine or the pharmaceutically acceptable salt thereof can be 0.001 ~ 10 weight% based on the total weight of the composition.

The content range of efinaconazole or the pharmaceutically acceptable salt thereof and the content range of octenidine or the pharmaceutically acceptable salt thereof overlap the ranges in the pharmaceutical composition for preventing or treating Tinea unguium, and thus they are omitted.

Since the pharmaceutical composition for preventing or treating Tinea corporis of the present invention can sterilize fungi and bacteria strongly by a complex antibacterial action, it exhibits a very excellent antibiotic effect compared to each single agent.

The pharmaceutical composition for preventing or treating Tinea corporis of the present invention can further include an additive.

Since the additive is the same as in the pharmaceutical composition for preventing or treating Tinea unguium, the overlapping description is omitted.

The pharmaceutical composition can further include a surfactant, a fragrance, a dye, a viscosity regulator, and the like for improved feeling of use and stability of the formulation, and can be applied in the same manner as in the above-mentioned pharmaceutical composition for preventing or treating Tinea unguium.

In an embodiment of the present invention, the pharmaceutical composition for preventing or treating Tinea corporis can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, 1,2-hexanediol), 1 ~ 20 weight% of a second

penetrant (eg, methylpropanediol), 0.01 ~ 3 weight% of a pH modifier (eg, succinic acid), and 1 ~ 95 weight% of a vehicle.

In another embodiment of the present invention, the pharmaceutical composition for preventing or treating Tinea corporis can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, 1,2-hexanediol), 1 ~ 20 weight% of a second penetrant (eg, methylpropanediol), 1 ~ 15 weight% of a film-former (eg, povidone), 0.01 ~ 3 weight% of a pH modifier (eg, succinic acid), and 1 ~ 95 weight% of a vehicle.

In another embodiment of the present invention, the pharmaceutical composition for preventing or treating Tinea corporis can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, dipropylene glycol), 1 ~ 20 weight% of a second penetrant (eg, methylpropanediol), 0.01 ~ 3 weight% of a pH modifier (eg, succinic acid), and 1 ~ 95 weight% of a vehicle.

In another embodiment of the present invention, the pharmaceutical composition for preventing or treating Tinea corporis can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, dipropylene glycol), 1 ~ 20 weight% of a second penetrant (eg, methylpropanediol), 1 ~ 15 weight% of a film-former (eg, povidone), 0.01 ~ 3 weight% of a pH modifier (eg, succinic acid), and 1 ~ 95 weight% of a vehicle.

The pH of the pharmaceutical composition for preventing or treating Tinea corporis can be 2 ~ 10.

The surface tension of the pharmaceutical composition for preventing or treating Tinea corporis can be 1 ~ 75 dyn/cm.

The water content of the pharmaceutical composition for preventing or treating Tinea corporis can be 0.001 ~ 95%.

The specific ranges are omitted because they overlap with the ranges in the pharmaceutical composition for preventing or treating Tinea unguium.

The daily dosage of the pharmaceutical composition for preventing or treating Tinea corporis can be 0.0001 ~ 300 g/day.

The pharmaceutical composition for preventing or treating Tinea corporis can be formulated as a dosage form selected from the group consisting of solutions, ointments, lotions, creams, gels, emulsions, suspensions, powder compacts, pastes, sticks, plasters, patches, tablets, powders, granules, capsules, gums, troches, mucoadhesives, inhalants, microcapsules, detergents, baths, injections, suppositories, liposomes and sprays.

In addition, the pharmaceutical composition for preventing or treating Tinea corporis can be formulated in the form of inhalation or oral preparations, parenteral preparations including external preparations, and injections.

The pharmaceutical composition for preventing or treating Tinea corporis is colorless or pale yellow, and can maintain the same color even after storage at 65°C for at least 5 weeks. That is, the pharmaceutical composition can retain the color of the composition when it was first formulated even after storage at 65°C for at least 5 weeks.

The density of the pharmaceutical composition for preventing or treating Tinea corporis can be 0.001 ~ 2 g/cm$^3$.

On the other hand, when the pharmaceutical composition for preventing or treating Tinea corporis is prepared as an external preparation and applied topically, a method of applying, spraying or attaching the composition to the affected site of a patient in an appropriate amount can be used.

In addition, the present invention provides a pharmaceutical composition for preventing or treating green nail syndrome, comprising efinaconazole or a pharmaceutically acceptable salt thereof; and octenidine or a pharmaceutically acceptable salt thereof as active ingredients.

On the other hand, green nail syndrome (GNS) is an infectious disease caused by the Gram-negative bacteria Pseudomonas aeruginosa, which shows black-green or blue-green discoloration of the nails. GNS mainly occurs in people who have jobs that come into contact with water a lot. If diseases such as paronychia, onycholysis, onychoptosis and onychodystrophy are accompanied, the probability of developing GNS increases.

Hereinafter, the detailed description of the pharmaceutical composition for preventing or treating green nail syndrome overlaps with the description of the pharmaceutical composition for preventing or treating Tinea unguium, and thus the description thereof is omitted.

The content of efinaconazole or the pharmaceutically acceptable salt thereof and the content of octenidine or the pharmaceutically acceptable salt thereof in the pharmaceutical composition for preventing or treating green nail syndrome can be applied in the same amount as in the pharmaceutical composition for preventing or treating Tinea unguium.

The content of efinaconazole or the pharmaceutically acceptable salt thereof can be 0.001 ~ 20 weight% based on the total weight of the composition.

The content of octenidine or the pharmaceutically acceptable salt thereof can be 0.001 ~ 10 weight% based

on the total weight of the composition.

The content range of efinaconazole or the pharmaceutically acceptable salt thereof and the content range of octenidine or the pharmaceutically acceptable salt thereof overlap the ranges in the pharmaceutical composition for preventing or treating Tinea unguium, and thus they are omitted.

Since the pharmaceutical composition for preventing or treating green nail syndrome of the present invention can sterilize fungi and bacteria strongly by a complex antibacterial action, it exhibits a very excellent antibiotic effect compared to each single agent.

The pharmaceutical composition for preventing or treating green nail syndrome of the present invention can further include an additive.

Since the additive is the same as in the pharmaceutical composition for preventing or treating Tinea unguium, the overlapping description is omitted.

The pharmaceutical composition can further include a surfactant, a fragrance, a dye, a viscosity regulator, and the like for improved feeling of use and stability of the formulation, and can be applied in the same manner as in the above-mentioned pharmaceutical composition for preventing or treating Tinea unguium.

In an embodiment of the present invention, the pharmaceutical composition for preventing or treating green nail syndrome can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, 1,2-hexanediol), 1 ~ 20 weight% of a second penetrant (eg, methylpropanediol), 0.01 ~ 3 weight% of a pH modifier (eg, succinic acid), and 1 ~ 95 weight% of a vehicle.

In another embodiment of the present invention, the pharmaceutical composition for preventing or treating green nail syndrome can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, 1,2-hexanediol), 1 ~ 20 weight% of a second penetrant (eg, methylpropanediol), 1 ~ 15 weight% of a film-former (eg, povidone), 0.01 ~ 3 weight% of a pH modifier (eg, succinic acid), and 1 ~ 95 weight% of a vehicle.

In another embodiment of the present invention, the pharmaceutical composition for preventing or treating green nail syndrome can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, dipropylene glycol), 1 ~ 20 weight% of a second penetrant (eg, methylpropanediol), 0.01 ~ 3 weight% of a pH modifier (eg, succinic acid), and 1 ~ 95 weight% of a vehicle.

In another embodiment of the present invention, the pharmaceutical composition for preventing or treating green nail syndrome can be formulated in the form of a solution containing 0.001 ~ 20 weight% of efinaconazole, 0.001 ~ 10 weight% of octenidine dihydrochloride, 1 ~ 90 weight% of a solvent (eg, ethanol), 0.001 ~ 3 weight% of an antioxidant, 0.0001 ~ 3 weight% of a chelating agent, 1 ~ 20 weight% of a first penetrant (eg, dipropylene glycol), 1 ~ 20 weight% of a second penetrant (eg, methylpropanediol), 1 ~ 15 weight% of a film-former (eg, povidone), 0.01 ~ 3 weight% of a pH modifier (eg, succinic acid), and 1 ~ 95 weight% of a vehicle.

The pH of the pharmaceutical composition for preventing or treating green nail syndrome can be 2 ~ 10.

The surface tension of the pharmaceutical composition for preventing or treating green nail syndrome can be 1 ~ 75 dyn/cm.

The water content of the pharmaceutical composition for preventing or treating green nail syndrome can be 0.001 ~ 95%.

The specific ranges are omitted because they overlap with the ranges in the pharmaceutical composition for preventing or treating Tinea unguium.

The daily dosage of the pharmaceutical composition for preventing or treating green nail syndrome can be 0.0001 ~ 300 g/day.

The pharmaceutical composition for preventing or treating green nail syndrome can be formulated as a dosage form selected from the group consisting of solutions, ointments, lotions, creams, gels, emulsions, suspensions, powder compacts, pastes, sticks, plasters, patches, tablets, powders, granules, capsules, gums, troches, mucoadhesives, inhalants, microcapsules, detergents, baths, injections, suppositories, liposomes and sprays.

In addition, the pharmaceutical composition for preventing or treating green nail syndrome can be formulated in the form of inhalation or oral preparations, parenteral preparations including external preparations, and injections.

The pharmaceutical composition for preventing or treating green nail syndrome is colorless or pale yellow, and can maintain the same color even after storage at 65°C for at least 5 weeks. That is, the pharmaceutical composition can retain the color of the composition when it was first formulated even after storage at 65°C for at least 5 weeks.

The density of the pharmaceutical composition for preventing or treating green nail syndrome can be 0.001 ~ 2 g/cm$^3$.

On the other hand, when the pharmaceutical composition for preventing or treating green nail syndrome is prepared as an external preparation and applied topically, a method of applying, spraying or attaching the composition to the affected site of a patient in an appropriate amount can be used.

The present inventors measured the susceptibility of the compositions comprising efinaconazole or a pharmaceutically acceptable salt thereof; and octenidine or a pharmaceutically acceptable salt thereof as active ingredients of the present invention (Examples 1 ~ 31) to 6 test strains (Trichophyton rubrum, Trichophyton mentagrophytes, Aspergillus niger, Candida albicans, Pseudomonas aeruginosa and Staphylococcus aureus) and 4 strains isolated from patients with Tinea unguium and green nail syndrome (Trichophyton rubrum, Trichophyton mentagrophytes, Candida albicans and Pseudomonas aeruginosa). As a result, it was confirmed that the composition of the present invention had a significantly increased antifungal (Trichophyton rubrum, Trichophyton mentagrophytes, Aspergillus niger and Candida albicans) effect compared to the efinaconazole single-component preparation of Comparative Example 1 and the octenidine single-component preparation of Comparative Example 2. It was also confirmed that the composition of the present invention had an excellent antibacterial effect against the Gram-negative bacteria Pseudomonas aeruginosa and the Gram-positive bacteria Staphylococcus aureus (Tables 5, 6, 7, 8 and 9).

Candida albicans and Staphylococcus aureus are known to be associated with Tinea unguium and cause inflammation around the nail. Therefore, the composition of the present invention was not only effective in the treatment of Tinea and green nail syndrome, but also effective in the treatment of nail-related diseases such as paronychia, onycholysis, onycholysis and onychodystrophy caused by inflammation around the nail.

The present inventors confirmed that the composition of the present invention has high antifungal and antibacterial effects by in vitro tests. The present inventors also confirmed that the composition of the present invention has better therapeutic effect on Tinea unguium and green nail syndrome than the efinaconazole single-component preparation or the octenidine single-component preparation in the animal models of Tinea and green nail syndrome using guinea pigs (Figures 1, 2, 4 and 5) .

In addition, the permeability of efinaconazole was measured in human skin, cow hoof, and the nail isolated from the human body. As a result, it was confirmed that the composition of the present invention, a combination preparation containing efinaconazole and octenidine dihydrochloride, showed a higher permeation concentration than that of efinaconazole alone (Tables 10 and 11, and Figure 3). The permeability of octenidine was also measured. As a result, it was confirmed that the composition of the present invention exhibited a higher permeation concentration than that of octenidine alone (Tables 15 and 16, and Figure 6).

The above results indicate that the composition of the present invention can treat Tinea or green nail syndrome more quickly and efficiently than efinaconazole or octenidine alone.

In addition, the present inventors confirmed the changes in physical properties such as discoloration by measuring the absorbance after storing the composition of the present invention at 65°C for 5 weeks. As a result, it was confirmed that the thermal stability of the composition of the present invention was well maintained compared to that of efinaconazole or octenidine alone (Tables 12, 13, 14, 17, 18 and 19).

In addition, the antibacterial activity was evaluated according to the contents of octenidine and efinaconazole. As a result, it was confirmed that the antifungal and antibacterial effects on 6 test strains (Trichophyton rubrum, Trichophyton mentagrophytes, Aspergillus niger, Candida albicans, Pseudomonas aeruginosa and Staphylococcus aureus) were increased in the range of 0.001 ~ 20 weight% of efinaconazole and in the range of 0.001 ~ 10 weight% of octenidine compared to efinaconazole alone (Table 21).

As described above, the composition of the present invention has a wide range of antibiotic effects including antifungal and antibacterial effects, and has excellent drug permeability to the skin and nail, so it can be used as a therapeutic agent for Tinea unguium, Tinea corporis and green nail syndrome. In addition, since the thermal stability is maintained at high temperature and the changes in physical properties such as discoloration do not occur, it can be stored stably for a long time.

The pharmaceutical composition of the present invention is convenient in use because it can be applied directly to the affected area through a brush, cotton swab, gauze, cotton, roller, ball or spray without grinding the nail when applied to Tinea and/or green nail syndrome.

In addition, since the composition of the present invention does not contain a cyclomethicone component, it is possible to solve the harmful problem caused by cyclomethicone.

Hereinafter, the present invention will be described in detail by the following examples.

However, the following examples are only for illustrating the present invention, and the contents of the present invention are not limited thereto.

**<Examples 1-31>**

Solutions (Examples 1-31) containing efinaconazole and octenidine dihydrochloride were prepared according to the components and compositions shown in Tables 1, 2, 3 and 4. The content of each component shown in Tables

1, 2, 3 and 4 represents the weight% of the total solution.

In Comparative Example 1, only efinaconazole was dissolved in an excipient, and in Comparative Example 2, only octenidine dihydrochloride was dissolved in an excipient.

First, a stock solution was prepared by dissolving efinaconazole and octenidine dihydrochloride in ethanol. After putting the ethanol solution containing efinaconazole and octenidine dihydrochloride in a container at room temperature, 1,2-hexanediol, methylpropanediol, dipropylene glycol, glycerin, butylhydroxyanisole, disodium ethylenediaminetetraacetic acid, succinic acid, povidone and purified water were sequentially added. Ethanol was further added thereto to adjust the total weight% (100 w/w%), and the mixture was filtered with a 0.45 μm filter to prepare a clear solution. The pH of the prepared solution was 5 ~ 7. The surface tension of 50 grams of each solution was measured at room temperature (21 ~ 25°C) using a surface tensiometer (Easy Dyne K20, KRUESS). As a result, the density of the prepared solution was 0.7 ~ 0.95 g/cm$^3$, and the surface tension was 18 ~ 35 dyn/cm.

[Table 1]

| Constituent | Example (weight%) | | | | | | | | |
|---|---|---|---|---|---|---|---|---|---|
| Constituent | Comparative Example 1 | Comparative Example 2 | Example 1 | Example 2 | Example 3 | Example 4 | Example 5 | Example 6 | Example 7 |
| Efinaconazole | 10.00 | - | 10.00 | 10.00 | 10.00 | 10.00 | 10.00 | 10.00 | 10.00 |
| Octenidine dihydrochloride | - | 0.10 | 0.10 | 0.10 | 0.10 | 0.10 | 0.10 | 0.10 | 0.10 |
| 1,2-Hexanediol | 3.00 | 3.00 | 3.00 | 3.00 | 1.50 | 1.00 | - | 2.00 | 1.00 |
| Methylpropanediol | 1.00 | 1.00 | 1.00 | - | 1.50 | - | 1.00 | 2.00 | 3.00 |
| Glycerin | 4.00 | 4.00 | 4.00 | 4.00 | 4.00 | 4.00 | 4.00 | 4.00 | 4.00 |
| Butylhydroxyanisole | 0.10 | 0.10 | 0.10 | 0.10 | 0.10 | 0.10 | 0.10 | 0.10 | 0.10 |
| Disodium ethylenediaminetetraacetic acid | 0.0002 | 0.0002 | 0.000 2 | 0.000 2 | 0.000 2 | 0.0002 | 0.000 2 | 0.000 2 | 0.000 2 |
| succinic acid | 0.05 | 0.05 | 0.05 | 0.05 | 0.05 | 0.05 | 0.05 | 0.05 | 0.05 |
| Ethanol | 79.8498 | 89.749 8 | 79.7498 | 80.7498 | 80.7498 | 82.7498 | 82.7498 | 79.7498 | 79.7498 |
| Purified water | 2.00 | 2.00 | 2.00 | 2.00 | 2.00 | 2.00 | 2.00 | 2.00 | 2.00 |
| Total (%) | 100.00 | 100.00 | 100.0 0 | 100.0 0 | 100.0 0 | 100.00 | 100.0 0 | 100.0 0 | 100.0 0 |

[Table 2]

| Constituent | Example (weight%) | | | | | | | |
|---|---|---|---|---|---|---|---|---|
| 구성 성분 | Example 8 | Example 9 | Example 10 | Example 11 | Example 12 | Example 13 | Example 14 | Example 15 |
| Efinaconazo le | 10.00 | 10.00 | 10.00 | 10.00 | 10.00 | 10.00 | 10.00 | 10.00 |
| Octenidine dihydrochlo ride | 0.10 | 0.10 | 0.10 | 0.10 | 0.10 | 0.10 | 0.10 | 0.10 |
| 1,2-Hexanediol | 3.00 | 3.00 | - | 1.50 | 1.50 | - | 2.00 | 1.00 |
| Methylpropa nediol | 1.00 | - | 3.00 | 1.50 | 0.50 | 2.00 | 2.00 | 3.00 |
| Glycerin | 4.00 | 4.00 | 4.00 | 4.00 | 4.00 | 4.00 | 4.00 | 4.00 |
| Butylhydrox yanisole | 0.10 | 0.10 | 0.10 | 0.10 | 0.10 | 0.10 | 0.10 | 0.10 |
| Disodium ethylenedia minetetraac etic acid | 0.0002 | 0.0002 | 0.0002 | 0.0002 | 0.0002 | 0.0002 | 0.0002 | 0.0002 |
| Succinic acid | 0.05 | 0.05 | 0.05 | 0.05 | 0.05 | 0.05 | 0.05 | 0.05 |
| Povidone | 10.00 | 10.00 | 10.00 | 10.00 | 10.00 | 10.00 | 10.00 | 10.00 |
| Ethanol | 69.749 8 | 70.7498 | 70.7498 | 70.7498 | 71.7498 | 71.7498 | 69.7498 | 69.7498 |
| Purified water | 2.00 | 2.00 | 2.00 | 2.00 | 2.00 | 2.00 | 2.00 | 2.00 |
| Total (%) | 100.00 | 100.00 | 100.00 | 100.00 | 100.00 | 100.00 | 100.00 | 100.00 |

[Table 3]

| Constitue nt | Example (weight%) | | | | | | | |
|---|---|---|---|---|---|---|---|---|
| 구성 성분 | Example 16 | Example 17 | Example 18 | Example 19 | Example 20 | Example 21 | Example 22 | Example 23 |
| Efinacona zole | 10.00 | 10.00 | 10.00 | 10.00 | 10.00 | 10.00 | 10.00 | 10.00 |
| Octenidin e dihydroch loride | 0.10 | 0.10 | 0.10 | 0.10 | 0.10 | 0.10 | 0.10 | 0.10 |
| Dipropyle ne glycol | 3.00 | 3.00 | - | 1.50 | 1.50 | - | 2.00 | 1.00 |
| Methylpro panediol | 1.00 | - | 3.00 | 1.50 | 0.50 | 2.00 | 2.00 | 3.00 |
| Glycerin | 4.00 | 4.00 | 4.00 | 4.00 | 4.00 | 4.00 | 4.00 | 4.00 |
| Butylhydr oxyanisol e | 0.10 | 0.10 | 0.10 | 0.10 | 0.10 | 0.10 | 0.10 | 0.10 |
| Disodium ethylened iaminetet raacetic acid | 0.0002 | 0.0002 | 0.0002 | 0.0002 | 0.0002 | 0.0002 | 0.0002 | 0.0002 |
| Succinic acid | 0.05 | 0.05 | 0.05 | 0.05 | 0.05 | 0.05 | 0.05 | 0.05 |
| Ethanol | 79.7498 | 80.7498 | 80.7498 | 80.7498 | 81.7498 | 81.7498 | 79.7498 | 79.7498 |
| Purified water | 2.00 | 2.00 | 2.00 | 2.00 | 2.00 | 2.00 | 2.00 | 2.00 |
| Total (%) | 100.00 | 100.00 | 100.00 | 100.00 | 100.00 | 100.00 | 100.00 | 100.00 |

[Table 4]

| Constituen t | Example (weight%) | | | | | | | |
|---|---|---|---|---|---|---|---|---|
| 구성 성분 | Exampl e 24 | Example 25 | Example 26 | Example 27 | Example 28 | Example 29 | Example 30 | Example 31 |
| Efinaconaz ole | 10.00 | 10.00 | 10.00 | 10.00 | 10.00 | 10.00 | 10.00 | 10.00 |
| Octenidine dihydrochl oride | 0.10 | 0.10 | 0.10 | 0.10 | 0.10 | 0.10 | 0.10 | 0.10 |
| Dipropylen e glycol | 3.00 | 3.00 | - | 1.50 | 1.50 | - | 2.00 | 1.00 |
| Methylprop anediol | 1.00 | - | 3.00 | 1.50 | 0.50 | 2.00 | 2.00 | 3.00 |
| Glycerin | 4.00 | 4.00 | 4.00 | 4.00 | 4.00 | 4.00 | 4.00 | 4.00 |
| Butylhydro xyanisole | 0.10 | 0.10 | 0.10 | 0.10 | 0.10 | 0.10 | 0.10 | 0.10 |
| Disodium ethylenedi aminetetra acetic acid | 0.0002 | 0.0002 | 0.0002 | 0.0002 | 0.0002 | 0.0002 | 0.0002 | 0.0002 |
| Succinic acid | 0.05 | 0.05 | 0.05 | 0.05 | 0.05 | 0.05 | 0.05 | 0.05 |
| Povidone | 10.00 | 10.00 | 10.00 | 10.00 | 10.00 | 10.00 | 10.00 | 10.00 |
| Ethanol | 69.749 8 | 70.7498 | 70.7498 | 70.7498 | 71.7498 | 71.7498 | 69.7498 | 69.7498 |
| Purified water | 2.00 | 2.00 | 2.00 | 2.00 | 2.00 | 2.00 | 2.00 | 2.00 |
| Total (%) | 100.00 | 100.00 | 100.00 | 100.00 | 100.00 | 100.00 | 100.00 | 100.00 |

**<Experimental Example 1> Susceptibility test to evaluate antimicrobial activity**

In general, clinical treatment results can be predicted through susceptibility tests of antifungal and antibacterial agents. The test strains of the susceptibility test for the evaluation of antimicrobial activity are Trichophyton rubrum, Trichophyton mentagrophytes, Aspergillus niger, Candida albicans, Pseudomonas aeruginosa, Staphylococcus aureus, which are the causative microorganisms of superficial fungal infection and green nail syndrome. In order to evaluate the antimicrobial activity against the above-mentioned 6 strains of microorganisms, this experiment was conducted by a tube dilution technique. For the evaluation of antimicrobial activity, the minimum inhibition concentrations ($MIC_{90}$) of Comparative Examples 1 and 2 and Examples 1-31 for each microorganism were compared, and the results are shown in Tables 5, 6, 7, and 8. The minimum inhibition concentration test was performed according to the classification based on the CLSI (Clinical and Laboratory Standards Institute) standard. Particularly, Trichophyton rubrum, Trichophyton mentagrophytes and Aspergillus niger (fungi) were tested by the CLSI M38-A2:2008 test method, Candida albicans (yeast) was tested by the CLSI M27-A3:2008 test method, Pseudomonas aeruginosa (Gram-negative bacteria) and Staphylococcus aureus (Gram-positive bacteria) were tested by the CLSI M07-A10:2015 test method as follows.

**1) Pre-culture of test microorganisms**

① Trichophyton rubrum, Trichophyton mentagrophytes, Aspergillus niger: The microorganisms were smeared on OA (Oatmeal 6%, Agar 1.25%, pH 6.0) medium and incubated at 30±2°C for 4 ~ 5 days.

(2) Candida albicans: The microorganisms were inoculated on SDA (Sabouraud Dextrose Agar) medium and incubated at 3512°C for 20 - 24 hours.

③ Pseudomonas aeruginosa, Staphylococcus aureus: The microorganisms were inoculated on TSA (Tryptic Soy Agar) medium and incubated at 35±2°C for 16 - 24 hours.

**2) Preparation of test cell solution and spore suspension**

OI Trichophyton rubrum, Trichophyton mentagrophytes, Aspergillus niger: After buffering with 0.165 mol/L of MOPS (3-N-morpholinolinopropane sulfonic acid) in RPMI 1640 medium (Gibco), a spore suspension of (2~3)×$10^3$ CFU/ml was prepared and used as a test cell solution.

(2) Candida albicans: After buffering with 0.165 mol/L of MOPS (3-N-morpholinolinopropane sulfonic acid) in RPMI 1640 medium (Gibco), a spore suspension of $5.ox_{lo}2$ - 2.5×$10^3$ CFU/ml was prepared and used as a test cell solution.

③ Pseudomonas aeruginosa, Staphylococcus aureus: The microorganisms were diluted to 1×$10^6$ CFU/ml in CAMHB (Cation-Adjusted Muller-Hinton Broth) and used as a test cell solution.

**3) Inoculation of test cell solution**

① Trichophyton rubrum, Trichophyton mentagrophytes, Aspergillus niger: The test cell solution was inoculated into the wells in which the test solution was dispensed by concentration (1 ml/well). The wells each containing 0.2 ml of the test solution (Comparative Examples 1 and 2, Examples 1 - 31) in RPMI1640 medium (buffered with 0.165 mol/L of MOPS) were used as the negative control, and the wells each containing 0.1 ml of the test solution (Comparative Examples 1 and 2, Examples 1 ~ 31) and 0.1 ml of the

test cell solution in RPMI1640 medium (buffered with 0.165 mol/L of MOPS) were used as the positive control. The tubes treated with the test cell solution were incubated at 3512°C for 46 ~ 50 hours.

(2) Candida albicans: The test cell solution was inoculated into the wells in which the test solution was dispensed by concentration (0.9 ml/well). The wells each containing 1.0 ml of the test solution (Comparative Examples 1 and 2, Examples 1 - 31) in RPMI1640 medium (buffered with 0.165 mol/L of MOPS) were used as the negative control, and the wells each containing 0.1 ml of the test solution (Comparative Examples 1 and 2, Examples 1 - 31) and 0.9 ml of the test cell solution in RPMI1640 medium (buffered with 0.165 mol/L of MOPS) were used as the positive control. The tubes treated with the test cell solution were incubated at 3512°C for 24 ~ 48 hours.

③ Pseudomonas aeruginosa, Staphylococcus aureus: The test cell solution was inoculated into the wells in which the test solution was dispensed by concentration (1 ml/well). The wells each containing 2 ml of the test solution (Comparative Examples 1 and 2, Examples 1 ~ 31) in CAMHB (Cation-Adjusted Muller-Hinton Broth) medium were used as the negative control, and the wells each containing 1 ml of the test solution (Comparative Examples 1 and 2, Examples 1 ~ 31) and 1 ml of the test cell solution in CAMHB (Cation-Adjusted Muller-Hinton Broth) were used as the positive control. The tubes treated with the test cell solution were incubated at 3512°C for 16 ~ 24 hours.

**4) Result decision**

When the growth of cells in each tube was visually observed after incubation, the minimum concentration at which cells did not grow was considered as $MIC_{90}$ (Minimum Inhibition Concentration). After incubation, the cell growth should not be observed in the negative control group, and the cell growth should be observed in the positive control group. The results obtained by the antimicrobial activity tests are shown in Tables 5, 6, 7 and 8.

[Table 5]

| Strain | Minimum inhibition concentration ($MIC_{90}$, volume%) | | | | | | | | |
|---|---|---|---|---|---|---|---|---|---|
| | Comparative Example 1 | Comparative Example 2 | Example 1 | Example 2 | Example 3 | Example 4 | Example 5 | Example 6 | Example 7 |
| Trichophyton rubrum | 0.003 1 | 0.1682 | 0.0008 | 0.0009 | 0.0007 | 0.0006 | 0.0008 | 0.0005 | 0.0009 |
| Trichophyton mentagrophytes | 0.000 19 | 0.0978 | 0.0000 5 | 0.0000 7 | 0.0000 4 | 0.0000 6 | 0.00004 | 0.0000 4 | 0.00005 |
| Aspergillus niger | 0.012 7 | 0.0085 | 0.0024 | 0.0022 | 0.0025 | 0.0018 | 0.0027 | 0.0021 | 0.0022 |
| Candida albicans | 1.253 4 | 0.2128 | 0.1562 | 0.1698 | 0.1621 | 0.1576 | 0.1684 | 0.1697 | 0.1621 |
| Pseudomonas aeruginosa | 6.253 1 | 1.226 | 0.7812 | 0.7523 | 0.8014 | 0.7627 | 0.7794 | 0.7765 | 0.8013 |
| Staphylococcus aureus | 1.431 6 | 0.4927 | 0.1993 | 0.1872 | 0.1752 | 0.1883 | 0.2120 | 0.1638 | 0.2071 |

[Table 6]

| Strain | Minimum inhibition concentration ($MIC_{90}$, volume%) | | | | | | | |
|---|---|---|---|---|---|---|---|---|
| Strain | Example 8 | Example 9 | Example 10 | Example 11 | Exampl e 12 | Example 13 | Example 14 | Exampl e 15 |
| Trichophyto n rubrum | 0.0010 | 0.0008 | 0.0007 | 0.0007 | 0.0010 | 0.0009 | 0.0008 | 0.0006 |
| Trichophyto n mentagrophy tes | 0.00006 | 0.00006 | 0.00008 | 0.00006 | 0.0000 5 | 0.00007 | 0.00004 | 0.0000 6 |
| Aspergillus niger | 0.0022 | 0.0032 | 0.0028 | 0.0026 | 0.0024 | 0.0031 | 0.0021 | 0.0025 |
| Candida albicans | 0.1662 | 0.1852 | 0.2647 | 0.1658 | 0.1668 | 0.1657 | 0.1682 | 0.1783 |
| Pseudomonas aeruginosa | 0.8865 | 0.7756 | 0.7923 | 0.8048 | 0.6954 | 0.7975 | 0.7543 | 0.7642 |
| Staphylococ cus aureus | 0.2567 | 0.1885 | 0.2136 | 0.2247 | 0.1775 | 0.1966 | 0.2058 | 0.1823 |

[Table 7]

| Strain | Minimum inhibition concentration ($MIC_{90}$, volume%) | | | | | | | |
|---|---|---|---|---|---|---|---|---|
| | Example 16 | Example 17 | Example 18 | Example 19 | Example 20 | Example 21 | Example 22 | Exampl e 23 |
| Trichophyton rubrum | 0.0009 | 0.0007 | 0.0008 | 0.0010 | 0.0010 | 0.0008 | 0.0006 | 0.0007 |
| Trichophyton mentagrophytes | 0.00007 | 0.00006 | 0.00006 | 0.00008 | 0.00007 | 0.00005 | 0.00006 | 0.0000 4 |
| Aspergillus niger | 0.0027 | 0.0029 | 0.0021 | 0.0031 | 0.0028 | 0.0024 | 0.0031 | 0.0019 |
| Candida albicans | 0.1725 | 0.1814 | 0.2527 | 0.1638 | 0.1667 | 0.2321 | 0.1856 | 0.1932 |
| Pseudomonas aeruginosa | 0.7865 | 0.7754 | 0.7893 | 0.7965 | 0.5954 | 0.7245 | 0.7346 | 0.8112 |
| Staphylococcus aureus | 0.2367 | 0.1964 | 0.2159 | 0.2045 | 0.1879 | 0.1922 | 0.2098 | 0.1766 |

[Table 8]

| Strain | Minimum inhibition concentration ($MIC_{90}$, volume%) | | | | | | | |
|---|---|---|---|---|---|---|---|---|
| | Example 24 | Example 25 | Example 26 | Example 27 | Example 28 | Example 29 | Example 30 | Exampl e 31 |
| Trichophyton rubrum | 0.0009 | 0.0010 | 0.0007 | 0.0010 | 0.0008 | 0.0010 | 0.0008 | 0.0006 |
| Trichophyton mentagrophytes | 0.00005 | 0.00004 | 0.00007 | 0.00007 | 0.00006 | 0.00008 | 0.00006 | 0.0000 5 |
| Aspergillus niger | 0.0022 | 0.0026 | 0.0033 | 0.0024 | 0.0039 | 0.0035 | 0.0031 | 0.0028 |
| Candida albicans | 0.1647 | 0.1852 | 0.2712 | 0.1639 | 0.1677 | 0.2452 | 0.1956 | 0.1623 |
| Pseudomonas aeruginosa | 0.7867 | 0.7756 | 0.6987 | 0.8107 | 0.6677 | 0.8007 | 0.7678 | 0.7937 |
| Staphylococcus aureus | 0.2435 | 0.1885 | 0.2024 | 0.2156 | 0.1654 | 0.1766 | 0.1886 | 0.2012 |

As shown in Tables 5, 6, 7 and 8, the antimicrobial activity of the compositions of Examples 1~31 against nail fungi, Gram-positive and Gram-negative bacteria was significantly increased compared to that of the composition of Comparative Example 1 not containing octenidine dihydrochloride. The antimicrobial activity of the compositions of Examples 1~31 was 2.4~7 times superior against Trichophyton rubrum, Trichophyton mentagrophytes and Aspergillus niger, the causative bacteria of Tinea unguium, and 4.6~8 times superior against Candida albicans (yeast) to that of the composition of Comparative Example 1.

In addition, the antimicrobial activity of the compositions of Examples 1~31 against the Gram-negative bacteria Pseudomonas aeruginosa and the Gram-positive bacteria Staphylococcus aureus was increased 5.6~9.4 times compared to that of the composition of Comparative Example 1. In particular, the compositions of Examples 1~31 showed 7.1~10.5 times stronger antimicrobial activity against Pseudomonas aeruginosa, the causative microorganism of green nail syndrome, than the composition of Comparative Example 1, so the treatment effect thereof on Tinea unguium, Tinea corporis and green nail syndrome was excellent. The compositions of Examples 1 - 31 showed more than 7 times the antimicrobial effect of the composition of Comparative Example 1 on Candida albicans and Staphylococcus aureus, which are related to Tinea unguium and cause inflammation around the nail, respectively, so they were effective in treating other nail-related diseases such as paronychia (whitlow), onycholysis, onychoptosis, onychodystrophy and onychomadesis.

As shown in Table 5, the antimicrobial activity of the compositions of Examples 1~7 against nail fungi, Gram-positive and Gram-negative bacteria was significantly increased compared to that of the composition of Comparative Example 2 not containing efinaconazole. In particular, the composition of the present invention supplemented the antimicrobial activity of octenidine dihydrochloride alone against Pseudomonas aeruginosa, the causative microorganism of green nail syndrome, by containing efinaconazole in the composition, and the effect was significantly increased.

As mentioned above, since the pharmaceutical compositions of Examples 1 ~ 31 showed good susceptibility test results for the causative microorganisms of Tinea unguium, Tinea corporis and green nail syndrome, it is expected that the compositions can treat the above-mentioned causative microorganisms simultaneously, and show excellent clinical treatment results.

**<Experimental Example 2> In vitro susceptibility test to clinically isolated strains**

Susceptibility test was performed on the 4 strains (Trichophyton rubrum, Trichophyton mentagrophytes, Candida albicans, and Pseudomonas aeruginosa) isolated from 5 patients with Tinea unguium and green nail syndrome among patients who visited the hospital's dermatology department. The compositions of Comparative Examples 1 and 2, and Examples 1, 8, 16 and 24 were used for the susceptibility test. Identification of the isolated strain was carried out by microscopy and PCR-RFLP (Polymerase Chain Reaction - Restriction Fragment Length Polymorphism) analysis, and then, two for each strain were selected and performed according to the susceptibility test method of Experimental Example 1 described above. Each strain was pre-cultured, and the formed mycelium was removed using sterilized gauze and a 5 ml triangular glass funnel. The test cell solution and spore suspension were prepared, inoculated and cultured to observe the growth thereof, and the minimum inhibition concentration ($MIC_{90}$) was determined. The results are shown in Table 9.

[Table 9]

| Strain | Minimum inhibition concentration ($MIC_{90}$, volume%) | | | | | |
|---|---|---|---|---|---|---|
| | Compara tive Example 1 | Compara tive Example 2 | Example 1 | Example 8 | Exampl e 16 | Example 24 |
| Trichophyton rubrum | 0.0028 | 0.1433 | 0.0006 | 0.0008 | 0.0007 | 0.0009 |
| Trichophyton mentagrophytes | 0.00018 | 0.0975 | 0.00003 | 0.00005 | 0.0000 6 | 0.00009 |
| Candida albicans | 1.0261 | 0.2012 | 0.1243 | 0.1438 | 0.1643 | 0.1527 |
| Pseudomonas aeruginosa | 5.8436 | 0.9354 | 0.5426 | 0.6123 | 0.7627 | 0.7346 |

As shown in Table 9, the susceptibility of the compositions of Comparative Examples 1 and 2 and Examples 1, 8, 16 and 24 to the four strains (Trichophyton rubrum, Trichophyton mentagrophytes, Candida albicans, and Pseudomonas aeruginosa) isolated from patients with Tinea unguium and green nail syndrome showed a similar trend to the results of Experimental Example 1.

The susceptibility of the compositions of Examples 1, 8, 16 and 24 was superior to that of the composition of Example 1 by 3 ~ 4.6 times for Trichophyton rubrum, the causative microorganism of Tinea unguium, 2 ~ 6 times for Trichomenta gropites, and 6.2 ~ 8.3 times for Candida albicans. Therefore, it was confirmed that the composition of the present invention was very effective in treating Tinea unguium and Tinea corporis. In addition, the compositions of Examples 1, 8, 16 and 24 showed a very strong and excellent therapeutic effect on Pseudomonas aeruginosa, the causative microorganism of green nail syndrome, by 7.7 ~ 10.8 times compared to the composition of Comparative Example 1.

That is, the therapeutic effect of the mixed formulation of efinaconazole and octenidine dihydrochloride was much more effective than that of the efinaconazole single-component preparation.

The susceptibility of the composition of Example 1 was superior to that of the composition of Comparative Example 2 not containing efinaconazole by 239 times for Trichophyton rubrum, the causative microorganism of Tinea unguium, 78 times for Trichomenta gropites, and 1.62 times for Candida albicans. Therefore, it was confirmed that the composition of the present invention was very effective in treating Tinea unguium and Tinea corporis.

In addition, the composition of Example 1 showed a very strong and excellent therapeutic effect on Pseudomonas aeruginosa, the causative microorganism of green nail syndrome, by 1.7 times compared to the composition of Comparative Example 2 not containing efinaconazole. That is, the therapeutic effect of the mixed formulation of efinaconazole and octenidine dihydrochloride was much more effective than that of the octenidine dihydrochloride single-component preparation.

As mentioned above, since the pharmaceutical compositions of Examples 1 ~ 31 showed good susceptibility test results for the causative microorganisms of Tinea unguium, Tinea corporis and green nail syndrome, it is expected that the compositions can treat the above-mentioned causative microorganisms simultaneously, and show excellent clinical treatment results.

**<Experimental Example 3> Evaluation of in vivo antimicrobial activity**

**(1) Trichophyton mentagrophytes**

The major causative microorganism of Tinea unguium is known as Trichophyton rubrum and Trichophyton mentagrophytes. The compounds of Comparative Examples 1 and 2, and Examples 1, 8, 16 and 24 were used as samples to evaluate the in vivo antimicrobial activity against the above microorganism, and a guinea pig infection model was used. Since Trichophyton rubrum is human-friendly, it is difficult to infect animals, so it was excluded from the animal model test, and Trichophyton mentagrophytes (ATCC 18748), which is animal-friendly and capable of infecting humans, was applied.

The test animals were male guinea pigs weighing 400 - 600 g. Guinea pigs were divided into 3 treatment groups with 3 animals per group. Trichophyton mentagrophytes was smeared on OA medium (Oatmeal 6%, Agar 1.25%, pH 6.0), pre-cultured at 30:t2°C for 4 ~ 5 days, and buffered in RPMI 1640 medium (Gibco) with 0.165 mol/L of MOPS (3-N-morpholinoprpane sulfonic acid). A spore suspension of $1\times10^7$ CFU/ml was prepared and used as a test cell solution. Infection was induced with sterile gauze moistened with 100 uL of the Trichophyton mentagrophytes spore suspension on 3 hind claws of each guinea pig, changing once every two days and sealing it up for 4 weeks.

30 uL of each composition of Comparative Examples 1 and 2, and Examples 1, 8, 16 and 24 of the present invention was topically applied with a nail brush once a day for 4 weeks. Infected/untreated groups were considered as the negative control. After treatment for 4 weeks, the animals were sacrificed. Then, the claws were collected, homogenized in phosphate buffered saline containing 0.25% (w/v) trypsin and 10 mm mol/L of $FeCl_2$, and then digested at 37°C for 1 hour. 100 uL of the homogenized solution was inoculated on OA medium containing 200 ug/ml of cycloheximide and 20 ug/ml of chloramphenicol, and cultured at 30°C for 14 days. After culturing, colonies of live fungi were counted, and the treatment effect was confirmed by the mean value of the number of viable cells (Log CFU/nail) and standard deviation. The results are shown in Figures 1 and 4.

Figure 1 is a graph showing the results of *in vivo* antifungal experiments according to Comparative Example 1 and various Examples. As shown in Figure 1, the antifungal effect of the compositions of Comparative Example 1 and Examples 1, 8, 16 and 24 on Trichophyton mentagrophytes, the causative microorganism of Tinea unguium, was increased 5 to 13 times, compared to before the treatment. The antimicrobial activity of the compositions of Examples 1, 8 16 and 24 was increased 2.6~3.3 times compared to that of the composition of Comparative Example 1. Therefore, it was confirmed that the compositions of Examples 1, 8, 16 and 24 composed of the mixed formulation of efinaconazole and octenidine dihydrochloride were much more effective than that of the composition of Comparative Example 1, the efinaconazole single-component preparation. Also, the antifungal effect of efinaconazole on Tinea unguium was increased due to the combination of efinaconazole and octenidine dihydrochloride. Through the results of Figure 1, it was confirmed that the pharmaceutical compositions according to various Examples of the present invention are very effective in the treatment of Tinea unguium and Tinea corporis.

Figure 4 is a graph showing the results of *in vivo* antifungal experiments according to Example 1 and Comparative Example 2. As shown in Figure 4, it was confirmed that the composition of Example 1 had a significantly superior antifungal effect compared to the composition of Comparative Example 2, the octenidine dihydrochloride single-component preparation.

**(2) Pseudomonas aeruginosa**

In order to confirm the antimicrobial activity against Pseudomonas aeruginosa, the causative microorganism of green nail syndrome accompanying Tinea unguium, the *in vivo* antimicrobial efficacy of the compositions of Comparative Examples 1 and 2, and Examples 1, 8, 16 and 24 were evaluated using a guinea pig infection model.

Pseudomonas aeruginosa (ATCC 9027) was applied as an infectious microorganism. The test animals were male guinea pigs weighing 400 ~ 600 g. Guinea pigs were divided into 3 treatment groups with 3 animals per group. Pseudomonas aeruginosa spore suspension was smeared on TSA (Tryptic Soy Agar) medium, and pre-cultured at 35±2°C for 16 ~ 24 hours. A spore suspension of $1\times10^7$ CFU/ml was prepared by diluting Pseudomonas aeruginosa in CAMHB (Cation-Adjusted Muller-Hinton Broth) medium and used as a test cell solution. Infection was induced with sterile gauze moistened with 100 uL of the Pseudomonas aeruginosa spore suspension on 3 hind claws of each guinea pig, changing once every two days and sealing it up for 4 weeks. 30 uL of each composition of Comparative Examples 1 and 2, and Examples 1, 8, 16 and 24 of the present invention was topically applied with a nail brush once a day for 4 weeks. Infected/untreated groups were considered as the negative control.

After treatment for 4 weeks, the animals were sacrificed. Then, the claws were collected, homogenized in phosphate buffered saline containing 0.25% (w/v) trypsin and 10 mm mol/L of $FeCl_2$, and then digested at 37 °C for 1 hour. 100 uL of the homogenized solution was inoculated on CAMHB (Cation-Adjusted Muller-Hinton Broth) medium containing 200 ug/ml of cycloheximide and 20 ug/ml of chloramphenicol, and cultured at 30°C for 14 days. After culturing, colonies of live Pseudomonas aeruginosa were counted, and the treatment effect was confirmed by the mean value of the number of viable cells (Log CFU/nail) and standard deviation. The results are shown in Figures 2 and 5.

Figure 2 is a graph showing the *in vivo* antibacterial effects according to Comparative Example 1 and various Examples. As shown in Figure 2, the antifungal effect of the compositions of Comparative Example 1 and Examples 1, 8, 16 and 24 on Pseudomonas aeruginosa, the causative microorganism of green nail syndrome, was increased 4 to 24 times, compared to before the treatment. The antimicrobial activity of the compositions of Examples 1, 8 16 and 24 was increased 3.3 ~ 6.5 times compared to that of the composition of Comparative Example 1. Therefore, it was confirmed that the compositions of Examples 1, 8, 16 and 24 composed of the mixed formulation of efinaconazole and octenidine dihydrochloride were much more effective than that of the composition of Comparative Example 1, the efinaconazole single-component preparation.

Figure 5 is a graph showing the *in vivo* antibacterial effects according to Example 1 and Comparative Example 2. As shown in Figure 5, it was confirmed that the composition of Example 1 had a significantly superior antimicrobial effect compared to the composition of Comparative Example 2, the octenidine dihydrochloride single-component preparation. That is, the composition of Example 1 composed of the mixed formulation of efinaconazole and octenidine dihydrochloride was much more effective than that of the composition of Comparative Example 2, the octenidine dihydrochloride single-component preparation.

**<Experimental Example 4> Evaluation of efinaconazole permeability and stability**

High-performance liquid chromatography (HPLC) was performed under the following conditions to analyze the content of efinaconazole.

* Analysis of the content of efinaconazole

- device: Agilent 1100 (DAD); concentration gradient according to time
- column: Inertsil ODS3V (250×4.6mm, 5.0μm)
- column temperature: 40°C
- moving phase: ⓐⓐ buffer ® acetonitrile:methanol (50:50 volume%)
- flow rate: 1.0ml/minute
- detector: UV detector
- detection wavelength: 210 nm
- sample injection amount: 10.0 ul
- sample concentration: 0.3mg/ml

* @ buffer preparation: After dissolving 1.36 g of $KH_2PO_4$ and 2.16 g of 1- octanesulphonic acid sodium in 1,000

ml of purified water, the pH was adjusted to 2.5, followed by filtration with a 0.45 μm membrane filter and degassing to prepare a buffer.

** efinaconazole content (weight%) = area of efinaconazole in sample x weight of standard material (mg) x 100 / area of efinaconazole in standard material x weight of sample (mg) x (100 - loss on drying of sample)

**<Experimental Example 4-1>** Evaluation of skin permeability using human skin

The skin permeability of efinaconazole to human skin was evaluated in a Franz diffusion cell using the compositions of Comparative Example 1, and Examples 1, 8, 16 and 24. HuSKin (Hans Biomed Co.) was used for the human skin. This was hydrated for 2 hours, 200 ul of each test sample was applied to the donor compartment, and 5 ml of phosphate-buffered saline (PBS) containing 1.0% (w/v) Tween 80 and 002% (w/v) kanamycin sulfate was filled in the acceptor compartment. At this time, the diffusion area was 1.13 $cm^2$. The sample collection amount was 200 ul for each measurement, and the solution in the acceptor compartment was supplemented with the same amount as the collection amount. The stirring speed was 600 rpm, the temperature was 37±0.5°C, and the number of repetitions per test was 3 times. Samples were collected every 24 hours and 48 hours. Since octenidine dihydrochloride was not included in the composition of Comparative Example 1, it was excluded from the content analysis test, and the content of efinaconazole in the obtained sample was analyzed by HPLC [Agilent 1100 (DAD)].

[Table 10]

| Sample | 24 hours | 48 hours |
|---|---|---|
| | **Permeated concentration** of efinaconazole (ug/ml) | **Permeated concentration** of efinaconazole (ug/ml) |
| Comparative Example 1 | 30.6512.12 | 48.67±1.26 |
| Example 1 | 54.78±1.75 | 3.6812.05 |
| Example 8 | 45.3211.02 | 72.46±1.46 |
| Example 16 | 50.2511.32 | 81.69±1.68 |
| Example 24 | 41.5211.54 | 70.78±1.39 |

As shown in Table 10, the compositions of Examples 1, 8, 16 and 24 exhibited higher permeated concentration of 1.4 ~ 1.8 times in 24-hour permeated concentration and 1.5 ~ 1.7 times in 48-hour permeated concentration compared to the composition of Comparative Example 1. Therefore, it was confirmed that the compositions of Examples 1, 8, 16 and 24 composed of the mixed formulation of efinaconazole and octenidine dihydrochloride were much more effective in treating Tinea unguium, Tinea corporis and green nail syndrome than the composition of Comparative Example 1, the efinaconazole single-component preparation.

**<Experimental Example 4-2>** Nail permeability test using cow hoofs

The permeability of efinaconazole to cow hoofs was evaluated using the compositions of Comparative Example 1, and Examples 1, 8, 16 and 24. The cow hoofs were cut into sections with a thickness of about 1.0 mm, inserted into a Franz diffusion cell, and hydrated for 2 hours. 200 ul of each test sample was applied to the donor compartment, and 5 ml of phosphate-buffered saline (PBS) containing 1.0% (w/v) Tween 80 and 0.002% (w/v) kanamycin sulfate was filled in the acceptor compartment. At this time, the diffusion area was 1.13 $cm^2$. The sample collection amount was 200 ul for each measurement, and the solution in the acceptor compartment was supplemented with the same amount as the collection amount. The stirring speed was 600 rpm, the temperature was 37±0.5°C, and the number of repetitions per

test was 3 times. Samples were collected every 24 hours and 48 hours. Since octenidine dihydrochloride was not included in the composition of Comparative Example 1, it was excluded from the content analysis test, and the content of efinaconazole in the obtained sample was analyzed by HPLC [Agilent 1100 (DAD)]. The results are shown in Table 11.

[Table 11]

| Sample | 24 hours | 48 hours |
|---|---|---|
| | **Permeated concentration** of efinaconazole (ug/ml) | **Permeated concentration** of efinaconazole (ug/ml) |
| Comparative Example 1 | 5.24±0.24 | 6.02±0.38 |
| Example 1 | 8.65±0.23 | 9.83±0.17 |
| Example 8 | 8.02±0.47 | 8.91±0.57 |
| Example 16 | 8.36±0.58 | 9.78±0.21 |
| Example 24 | 7.8810.28 | 8.82±0.36 |

As shown in Table 11, the compositions of Examples 1, 8, 16 and 24 exhibited higher permeated concentration of 1.5 ~ 1.7 times in 24-hour permeated concentration and 1.5 ~ 1.7 times in 48-hour permeated concentration compared to the composition of Comparative Example 1. Therefore, it was confirmed that the compositions of Examples 1, 8, 16 and 24 composed of the mixed formulation of efinaconazole and octenidine dihydrochloride were much more effective in treating Tinea unguium, Tinea corporis and green nail syndrome than the composition of Comparative Example 1, the efinaconazole single-component preparation.

**<Experimental Example 4-3>** Evaluation of nail permeability in human body

The nails cut from a healthy adult human body were obtained, washed with PBS, and hydrated in physiological saline for 2 hours. The hydrated nails were inserted into a Franz diffusion cell. 100 ul of each sample (compositions of Comparative Example 1, and Examples 1, 8, 16 and 24) was applied to the donor compartment once a day for 10 days, and 200 ul of the sample was collected from the acceptor compartment. 5 ml of phosphate-buffered saline (PBS) containing 1.0% (w/v) Tween 80 and 0.002% (w/v) kanamycin sulfate was filled in the acceptor compartment. At this time, the diffusion area was 1.13 $cm^2$. The sample collection amount was 200 ul for each measurement, and the solution in the acceptor compartment was supplemented with the same amount as the collection amount. The stirring speed was 600 rpm, the temperature was 32±0.5°C, and the number of repetitions per test was 3 times. Samples were collected at 1, 3, 7, 13, 21 and 24 hours on day 1, and every 48 hours from day 2 to day 10. Meanwhile, the cumulative permeated concentration of octenidine dihydrochloride was not included in Comparative Example 1, so it was excluded from the content analysis test. The content of efinaconazole in the obtained sample was analyzed by HPLC [Agilent 1100 (DAD)]. The obtained results were confirmed as mean value of the cumulative permeated concentration (ug/ml) and standard deviation. The results are shown in Figure 3.

Figure 3 is a graph showing the permeated concentration of efinaconazole accumulated for 10 days according to Comparative Example 1 and various Examples. As shown in Figure 3, the compositions of Examples 1, 8, 16 and 24 exhibited higher permeated concentration of 1.5 ~ 1.7 times in the 1$^{st}$ day permeated concentration, 1.3 ~ 1.7 times in the 2$^{nd}$ day cumulative permeated concentration, 1.4 ~ 1.6 times in the 6$^{th}$ day cumulative permeated concentration, and 1.4 ~ 1.5 times in the 10$^{th}$ day cumulative permeated concentration compared to the composition of Comparative Example 1. Therefore, it was confirmed that the compositions of Examples 1, 8, 16 and 24 composed of the mixed formulation of efinaconazole and octenidine dihydrochloride were much more effective in treating Tinea unguium, Tinea corporis and green nail syndrome than the composition of Comparative Example 1, the efinaconazole single-component preparation.

**<Experimental Example 4-4> Stability test**

The compositions of Comparative Example 1 (efinaconazole single-component preparation), and Example 1 ~ 31 (mixed formulation of efinaconazole and octenidine dihydrochloride) were put into 20 ml transparent vials (10 ml/vial), which were sealed and stored at 65°C for 5 weeks (35 days). UV/VIS absorption spectra of the samples at the initial stage and after 5 weeks at 65°C were measured. The absorbance was measured at various wavelengths (400 nm, 500 nm and 600 nm) using a Mega-800 spectrophotometer (Scinco, Korea). The cutoff values of the absorbance

were 0.3 AU (absorbance unit) at 400 nm, 0.1 AU at 500 nm, and 0.1 AU or less at 600 nm. The results are shown in Tables 12, 13 and 14.

[Table 12]

| Absorbance at 400 nm | | |
|---|---|---|
| Sample | Initial stage | After 5 weeks |
| Comparative Example 1 | 0.106±0.003 | 0.125±0.003 |
| Comparative Example 2 | 0.029±0.002 | 0.034±0.001 |
| Example 1 | 0.078±0.002 | 0.083±0.002 |
| Example 2 | 0.075±0.001 | 0.082±0.003 |
| Example 3 | 0.072±0.001 | 0.080±0.003 |
| Example 4 | 0.081±0.002 | 0.087±0.001 |
| Example 5 | 0.077±0.003 | 0.085±0.001 |
| Example 6 | 0.080±0.002 | 0.089±0.001 |
| Example 7 | 0.076±0.001 | 0.084±0.001 |
| Example 8 | 0.079±0.003 | 0.085±0.002 |
| Example 9 | 0.078±0.002 | 0.083±0.001 |
| Example 10 | 0.076±0.003 | 0.082±0.001 |
| Example 11 | 0.081±0.001 | 0.088±0.002 |
| Example 12 | 0.077±0.002 | 0.084±0.003 |
| Example 13 | 0.079±0.001 | 0.086±0.002 |
| Example 14 | 0.078±0.001 | 0.088±0.002 |
| Example 15 | 0.080±0.002 | 0.086±0.003 |
| Example 16 | 0.068±0.002 | 0.077±0.003 |
| Example 17 | 0.069±0.002 | 0.078±0.001 |
| Example 18 | 0.070±0.001 | 0.079±0.002 |
| Example 19 | 0.071±0.002 | 0.076±0.003 |
| Example 20 | 0.067±0.002 | 0.075±0.001 |
| Example 21 | 0.068±0.001 | 0.077±0.003 |
| Example 22 | 0.069±0.002 | 0.076±0.003 |
| Example 23 | 0.070±0.001 | 0.079±0.002 |
| Example 24 | 0.091±0.001 | 0.097±0.002 |
| Example 25 | 0.089±0.002 | 0.097±0.002 |
| Example 26 | 0.093±0.001 | 0.10010.003 |
| Example 27 | 0.090±0.002 | 0.098±0.002 |
| Example 28 | 0.091±0.002 | 0.10010.003 |
| Example 29 | 0.090±0.001 | 0.097±0.002 |
| Example 30 | 0.092±0.002 | 0.10010.003 |
| Example 31 | 0.091±0.002 | 0.098±0.001 |

[Table 13]

| Absorbance at 500 nm | | |
|---|---|---|
| Sample | Initial stage | After 5 weeks |
| Comparative Example 1 | 0.080±0.002 | 0.094±0.001 |
| Comparative Example 2 | 0.028±0.001 | 0.033±0.001 |
| Example 1 | 0.046±0.001 | 0.050±0.002 |
| Example 2 | 0.047±0.002 | 0.052±0.001 |
| Example 3 | 0.045±0.001 | 0.051±0.002 |
| Example 4 | 0.048±0.003 | 0.054±0.001 |
| Example 5 | 0.048±0.003 | 0.053±0.002 |
| Example 6 | 0.045±0.003 | 0.051±0.001 |
| Example 7 | 0.046±0.001 | 0.051±0.003 |
| Example 8 | 0.044±0.002 | 0.050±0.002 |
| Example 9 | 0.045±0.002 | 0.050±0.003 |
| Example 10 | 0.044±0.003 | 0.052±0.001 |
| Example 11 | 0.046±0.003 | 0.051±0.003 |
| Example 12 | 0.046±0.002 | 0.053±0.001 |
| Example 13 | 0.045±0.001 | 0.052±0.003 |
| Example 14 | 0.046±0.002 | 0.052±0.003 |
| Example 15 | 0.047±0.001 | 0.054±0.003 |
| Example 16 | 0.043±0.002 | 0.050±0.002 |
| Example 17 | 0.042±0.003 | 0.050±0.002 |
| Example 18 | 0.045±0.002 | 0.052±0.003 |
| Example 19 | 0.044±0.001 | 0.051±0.003 |
| Example 20 | 0.045±0.003 | 0.052±0.002 |
| Example 21 | 0.047±0.001 | 0.055±0.002 |
| Example 22 | 0.047±0.002 | 0.054±0.001 |
| Example 23 | 0.046±0.001 | 0.052±0.003 |
| Example 24 | 0.055±0.003 | 0.061±0.002 |
| Example 25 | 0.057±0.002 | 0.063±0.003 |
| Example 26 | 0.054±0.003 | 0.061±0.002 |
| Example 27 | 0.056±0.003 | 0.062±0.002 |
| Example 28 | 0.057±0.001 | 0.064±0.002 |
| Example 29 | 0.055±0.001 | 0.062±0.003 |
| Example 30 | 0.056±0.002 | 0.063±0.001 |
| Example 31 | 0.055±0.001 | 0.062±0.002 |

[Table 14]

| Absorbance at 600 nm | | |
|---|---|---|
| Sample | Initial stage | After 5 weeks |
| Comparative Example 1 | 0.072±0.002 | 0.088±0.002 |
| Comparative Example 2 | 0.027±0.001 | 0.032±0.002 |
| Example 1 | 0.037±0.002 | 0.040±0.001 |
| Example 2 | 0.037±0.001 | 0.043±0.002 |
| Example 3 | 0.039±0.003 | 0.046±0.001 |
| Example 4 | 0.038±0.002 | 0.042±0.003 |
| Example 5 | 0.037±0.001 | 0.044±0.002 |
| Example 6 | 0.038±0.001 | 0.042±0.001 |
| Example 7 | 0.037±0.002 | 0.046±0.002 |
| Example 8 | 0.035±0.003 | 0.041±0.001 |
| Example 9 | 0.036±0.002 | 0.043±0.001 |
| Example 10 | 0.038±0.002 | 0.046±0.002 |
| Example 11 | 0.035±0.002 | 0.043±0.001 |
| Example 12 | 0.036±0.001 | 0.041±0.001 |
| Example 13 | 0.038±0.002 | 0.043±0.001 |
| Example 14 | 0.038±0.002 | 0.042±0.002 |
| Example 15 | 0.037±0.001 | 0.045±0.003 |
| Example 16 | 0.043±0.003 | 0.050±0.002 |
| Example 17 | 0.044±0.002 | 0.050±0.001 |
| Example 18 | 0.046±0.002 | 0.051±0.002 |
| Example 19 | 0.046±0.001 | 0.052±0.002 |
| Example 20 | 0.044±0.003 | 0.051±0.001 |
| Example 21 | 0.043±0.002 | 0.050±0.001 |
| Example 22 | 0.044±0.002 | 0.053±0.001 |
| Example 23 | 0.046±0.001 | 0.054±0.002 |
| Example 24 | 0.046±0.001 | 0.055±0.001 |
| Example 25 | 0.047±0.002 | 0.053±0.003 |
| Example 26 | 0.045±0.002 | 0.052±0.001 |
| Example 27 | 0.046±0.001 | 0.053±0.002 |
| Example 28 | 0.047±0.002 | 0.055±0.002 |
| Example 29 | 0.044±0.001 | 0.051±0.001 |
| Example 30 | 0.045±0.002 | 0.053±0.001 |
| Example 31 | 0.047±0.002 | 0.052±0.001 |

As shown in Tables 12 ~ 14, the compositions of Examples 1 ~ 31 showed about twice or more stability compared to the composition of Comparative Example 1. In addition, through the results of the compositions of Examples 8 ~ 15 and the compositions of Examples 24 ~ 31, it was confirmed that there was no significant change in the content of the main component even when povidone was added.

**<Experimental Example 5>** Evaluation of octenidine permeability and stability

High-performance liquid chromatography (HPLC) was performed under the following conditions to analyze the content of octenidine dihydrochloride.

* Analysis of the content of octenidine dihydrochloride

- device: Agilent 1100 (DAD); concentration gradient according to time
- column: Zorbax Eclipse XDB-C8 (150×4.6mm, 5.0um)
- column temperature: 15.0°C
- moving phase: methanol
- detector: UV detector
- detection wavelength: 220 nm
- sample injection amount: 5.0 ul
- sample concentration: 0.1 mg/ml
- internal standard material: butylparaben

* octenidine dihydrochloride content (weight%)= amount of internal standard material x area of octenidine dihydrochloride x 100 x dilution factor / amount of sample x area of internal standard material

**<Experimental Example 5-1>** Evaluation of skin permeability using human skin

The skin permeability of octenidine dihydrochloride to human skin was evaluated in a Franz diffusion cell using the compositions of Example 1 and Comparative Example 2. HuSKin (Hans Biomed Co.) was used for the human skin. This was hydrated for 2 hours, 200 ul of each test sample was applied to the donor compartment, and 5 ml of phosphate-buffered saline (PBS) containing 1.0% (w/v) Tween 80 and 0.002% (w/v) kanamycin sulfate was filled in the acceptor compartment. At this time, the diffusion area was 1.13 $cm^2$. The sample collection amount was 200 ul for each measurement, and the solution in the acceptor compartment was supplemented with the same amount as the collection amount. The stirring speed was 600 rpm, the temperature was 37±0.5°C, and the number of repetitions per test was 3 times. Samples were collected every 24 hours and 48 hours. The content of octenidine dihydrochloride in the obtained sample was analyzed by HPLC [Agilent 1100 (DAD)]. The results are shown in Table 15.

[Table 15]

| Sample | 24 hours | 48 hours |
|---|---|---|
| | Permeated concentration of octenidine dihydrochloride (ug/ml) | Permeated concentration of octenidine dihydrochloride (ug/ml) |
| Example 1 | 2.22±0.17 | 3.88±0.21 |
| Comparative Example 2 | 1.45±0.11 | 2.74±0.18 |

As shown in Table 15, the composition of Example 1 exhibited higher permeated concentration of 1.5 times in 24-hour permeated concentration and 1.4 times in 48-hour permeated concentration compared to the composition of Comparative Example 2. Therefore, it was confirmed that the composition of Example 1 composed of the mixed formulation of efinaconazole and octenidine dihydrochloride was much more effective in treating green nail syndrome than the composition of Comparative Example 2, the octenidine dihydrochloride single-component preparation.

**<Experimental Example 5-2>** Nail permeability test using cow hoofs

The permeability of octenidine dihydrochloride to cow hoofs was evaluated using the compositions of Example

1 and Comparative Example 2. The cow hoofs were cut into sections with a thickness of about 1.0 mm, inserted into a Franz diffusion cell, and hydrated for 2 hours. 200 ul of each test sample was applied to the donor compartment, and 5 ml of phosphate-buffered saline (PBS) containing 1.0% (w/v) Tween 80 and 0.002% (w/v) kanamycin sulfate was filled in the acceptor compartment. At this time, the diffusion area was 1.13 $cm^2$. The sample collection amount was 200 ul for each measurement, and the solution in the acceptor compartment was supplemented with the same amount as the collection amount. The stirring speed was 600 rpm, the temperature was 37±0.5°C, and the number of repetitions per test was 3 times. Samples were collected every 24 hours and 48 hours. The content of octenidine dihydrochloride in the obtained sample was analyzed by HPLC [Agilent 1100 (DAD)]. The results are shown in Table 16.

[Table 16]

| Sample | 24 hours | 48 hours |
|---|---|---|
| | Permeated concentration of octenidine dihydrochloride (ug/ml) | Permeated concentration of octenidine dihydrochloride (ug/ml) |
| Example 1 | 0.45±0.03 | 0.83±0.04 |
| Comparative Example 2 | 0.32±0.02 | 0.51±0.03 |

As shown in Table 16, the composition of Example 1 exhibited higher permeated concentration of 1.4 in 24-hour permeated concentration and 1.6 times in 48-hour permeated concentration compared to the composition of Comparative Example 2. Therefore, it was confirmed that the composition of Example 1 composed of the mixed formulation of efinaconazole and octenidine dihydrochloride was much more effective in treating green nail syndrome than the composition of Comparative Example 2, the octenidine dihydrochloride single-component preparation.

**<Experimental Example 5-3>** Evaluation of nail permeability in human body

The nails cut from a healthy adult human body were obtained, washed with PBS, and hydrated in physiological saline for 2 hours. The hydrated nails were inserted into a Franz diffusion cell. 100 ul of each sample (compositions of Example 1 and Comparative Example 2) was applied to the donor compartment once a day for 10 days, and 200 ul of the sample was collected from the acceptor compartment. 5 ml of phosphate-buffered saline (PBS) containing 1.0% (w/v) Tween 80 and 0.002% (w/v) kanamycin sulfate was filled in the acceptor compartment. At this time, the diffusion area was 1.13 $cm^2$. The sample collection amount was 200 ul for each measurement, and the solution in the acceptor compartment was supplemented with the same amount as the collection amount. The stirring speed was 600 rpm, the temperature was 32±0.5°C, and the number of repetitions per test was 3 times. Samples were collected at 1, 3, 7, 13, 21 and 24 hours on day 1, and every 48 hours from day 2 to day 10. The content of octenidine dihydrochloride in the obtained sample was analyzed by HPLC [Agilent 1100 (DAD)]. The obtained results were confirmed as mean value of the cumulative permeated concentration (ug/ml) and standard deviation. The results are shown in Figure 6.

Figure 6 is a graph showing the permeated concentration of octenidine dihydrochloride accumulated for 10 days according to Example 1 and Comparative Example 2. As shown in Figure 6, the composition of Example 1 exhibited higher permeated concentration of 1.5 times in the $1^{st}$ day permeated concentration, 1.4 times in the $2^{nd}$ day cumulative permeated concentration, 1.6 times in the $6^{th}$ day cumulative permeated concentration, and 1.5 times in the $10^{th}$ day cumulative permeated concentration compared to the composition of Comparative Example 1. Therefore, it was confirmed that the composition of Example 1 composed of the mixed formulation of efinaconazole and octenidine dihydrochloride was much more effective in treating green nail syndrome than the composition of Comparative Example 2, the octenidine dihydrochloride single-component preparation.

**<Experimental Example 5-4> Stability test**

The compositions of Example 1 (mixed formulation of efinaconazole and octenidine dihydrochloride) and Comparative Example 2 (octenidine dihydrochloride single-component preparation) were put into 20 ml transparent vials (10 ml/vial), which were sealed and stored at 65°C for 5 weeks (35 days). UV/VIS absorption spectra of the samples at the initial stage and after 5 weeks at 65°C were measured. The absorbance was measured at various wavelengths (400 nm, 500 nm and 600 nm) using a Mega-800 spectrophotometer (Scinco, Korea). The cutoff values of the absorbance were 0.3 AU (absorbance unit) at 400 nm, 0.1 AU at 500 nm, and 0.1 AU or less at 600 nm. The results are shown in Tables 17, 18 and 19.

[Table 17]

| Absorbance at 400 nm | | |
|---|---|---|
| Sample | Initial stage | After 5 weeks |
| Example 1 | 0.078±0.002 | 0.083±0.002 |
| Comparative Example 2 | 0.029±0.002 | 0.034±0.001 |

[Table 18]

| Absorbance at 500 nm | | |
|---|---|---|
| Sample | Initial stage | After 5 weeks |
| Example 1 | 0.046±0.001 | 0.050±0.002 |
| Comparative Example 2 | 0.028±0.001 | 0.033±0.001 |

[Table 19]

| Absorbance at 600 nm | | |
|---|---|---|
| Sample | Initial stage | After 5 weeks |
| Example 1 | 0.037±0.002 | 0.040±0.001 |
| Comparative Example 2 | 0.027±0.001 | 0.032±0.002 |

As shown in Tables 17 ~ 19, the composition of Example 1 was very stable compared to the composition of Comparative Example 2, octenidine dihydrochloride single-component preparation. The absorbance at 400 nm of the composition of Example 1 was increased by 6.4% while the composition of Comparative Example 2 was increased by 17.2%. The absorbance at 500 nm of the composition of Example 1 was increased by 8.7% while the composition of Comparative Example 2 was increased by 17.9%. The absorbance at 600 nm of the composition of Example 1 was increased by 8.1% while the composition of Comparative Example 2 was increased by 18.5%. Therefore, it was confirmed that the stability of the composition of Example 1 composed of the mixed formulation of efinaconazole and octenidine dihydrochloride was superior to the composition of Comparative Example 2, the octenidine dihydrochloride single-component preparation.

**<Examples 32 ~ 43>**

The solutions of Examples 32 ~ 43 containing efinaconazole and octenidine dihydrochloride were prepared according to the components and compositions shown in Table 20. The content of each component indicated in Table 20 represents the weight% to the total solution. First, a stock solution was prepared by dissolving efinaconazole and octenidine dihydrochloride in ethanol. After putting the ethanol solution containing efinaconazole and octenidine dihydrochloride in a container at room temperature, 1,2-hexanediol, methylpropanediol, glycerin, butylhydroxyanisole, disodium ethylenediaminetetraacetic acid, succinic acid, and purified water were sequentially added. Ethanol was further added thereto to adjust the total weight% (100 w/w%), and the mixture was filtered with a 0.45 μm filter to prepare a clear solution. The pH of the prepared solution was 5 ~ 7. The surface tension of 50 grams of each solution was measured at room temperature (21 ~ 25°C) using a surface tensiometer (Easy Dyne K20, KRUESS). As a result, the density of the prepared solution was 0.7 ~ 0.95 g/cm$^3$, and the surface tension was 18 ~ 35 dyn/cm.

[Table 20]

| Constituent | Example (weight) | | | | | | | | | | | |
|---|---|---|---|---|---|---|---|---|---|---|---|---|
| | Example 32 | Example 33 | Example 34 | Example 35 | Example 36 | Example 37 | Example 38 | Example 39 | Example 40 | Example 41 | Example 42 | Example 43 |
| Efinaconazole | 0.001 | 10 | 20 | 0.005 | 0.01 | 0.05 | 0.1 | 0.5 | 1 | 3 | 5 | 15 |
| Octenidine dihydrochloride | 10 | 5 | 0.001 | 8 | 5 | 3 | 1 | 0.5 | 0.1 | 0.05 | 0.01 | 0.005 |
| 1,2-Hexanediol | 3.00 | 3.00 | 1.00 | 3 | 3 | 3 | 3 | 3 | 3 | 3 | 3 | 1 |
| Methylpropanediol | 1.00 | 1.00 | 1.00 | 1 | 1 | 1 | 1 | 1 | 1 | 1 | 1 | 1 |
| Glycerin | 4.00 | 4.00 | - | 4.00 | 4.00 | 4.00 | 4.00 | 4.00 | 4.00 | 4.00 | 4.00 | - |
| Buthylhydroxyanisole | 0.10 | 0.10 | 0.10 | 0.10 | 0.10 | 0.10 | 0.10 | 0.10 | 0.10 | 0.10 | 0.10 | 0.10 |
| Disodium ethylenediaminetetraacetic acid | 0.0002 | 0.0002 | 0.0002 | 0.0002 | 0.0002 | 0.0002 | 0.0002 | 0.0002 | 0.0002 | 0.0002 | 0.0002 | 0.0002 |
| Succinic acid | 0.05 | 0.05 | 0.05 | 0.05 | 0.05 | 0.05 | 0.05 | 0.05 | 0.05 | 0.05 | 0.05 | 0.05 |
| Ethanol | 79.8488 | 74.8498 | 75.8488 | 81.8448 | 84.8398 | 86.7998 | 88.7498 | 38.8498 | 83.7498 | 86.7998 | 84.8398 | 80.8448 |
| Purified water | 2.00 | 2.00 | 2.00 | 2.00 | 2.00 | 2.00 | 2.00 | 2.00 | 2.00 | 2.00 | 2.00 | 2.00 |
| Total (%) | 100.00 | 100.00 | 100.00 | 100.00 | 100.00 | 100.00 | 100.00 | 100.00 | 100.00 | 100.00 | 100.00 | 100.00 |

**<Experimental Example 6> Susceptibility test to evaluate antimicrobial activity**

Antimicrobial activity was evaluated in the same manner as described in Experimental Example 1. The minimum inhibition concentrations ($MIC_{90}$) of the compositions of Examples 32 ~ 43 for each microorganism were compared, and the results are shown in Table 21.

[Table 21]

| Strain | Minimum inhibition concentration ($MIC_{90}$, volume%) | | | | | | | | | | | |
|---|---|---|---|---|---|---|---|---|---|---|---|---|
| | Example 32 | Example 33 | Example | Example 34 35 | Example 36 | Example 37 | Example 38 | Example 39 | Example 40 | Example 41 | Example 42 | Example 43 |
| Trichophyton rubrum (T. rubrum) ATCC 28188 | 0.0012 | 0.00008 | 0.00003 | 0.0010 | 0.0008 | 0.0006 | 0.0003 | 0.0001 | 0.00026 | 0.00020 | 0.00015 | 0.00010 |
| Trichophyton mentagrophytes (T. mentagrophytes) ATCC 18748 | 0.0009 | 0.000007 | 0.000001 | 0.0004 | 0.0008 | 0.0007 | 0.0005 | 0.0003 | 0.0001 | 0.00015 | 0.00017 | 0.000008 |
| Aspergillus niger (A. niger) ATCC 9642 | 0.003 | 0.00009 | 0.00004 | 0.00002 | 0.00009 | 0.0002 | 0.0004 | 0.0007 | 0.0043 | 0.0090 | 0.020 | 0.050 |
| Candida albicans (C. albicans) ATCC 10231 | 0.0012 | 0.0043 | 0.012 | 0.0009 | 0.0012 | 0.0014 | 0.0015 | 0.0019 | 0.0024 | 0.0031 | 0.0040 | 0.0050 |
| Pseudomonas aeruginosa (P. aeruginosa) ATCC 9027 | 0.0024 | 0.0152 | 0.026 | 0.0011 | 0.0026 | 0.0038 | 0.0041 | 0.054 | 0.070 | 0.092 | 0.162 | 0.198 |
| Staphylococcus aureus (S. aureus) ATCC 6538 | 0.0015 | 0.00484 | 0.043 | 0.010 | 0.0015 | 0.0021 | 0.0036 | 0.012 | 0.046 | 0.095 | 0.176 | 0.258 |

As shown in Table 21, the antimicrobial activity of the compositions of Examples 32~43 against nail fungi, Gram-positive and Gram-negative bacteria was significantly increased compared to that of the composition of Comparative Example 1 not containing octenidine dihydrochloride. As mentioned above, since the pharmaceutical compositions of Examples 32 ~ 43 showed good susceptibility test results for the causative microorganisms of Tinea unguium, Tinea corporis and green nail syndrome, it is expected that the compositions can treat the above-mentioned causative microorganisms simultaneously, and show excellent clinical treatment results.

As described above, the composition of the present invention has an advantage in that it has excellent therapeutic effects on Tinea (for example, Tinea unguium and Tinea corporis), and can also treat green nail syndrome. In addition, the composition of the present invention has a much higher antifungal effect than the conventional efinaconazole single-component preparation, and has an excellent antibacterial effect against Gram-negative bacteria (eg, Pseudomonas aeruginosa) and Gram-positive bacteria (eg, Staphylococcus aureus), so that the composition can be applied to the prevention or treatment of at least one of Tinea unguium, Tinea corporis and green nail syndrome. Further, the composition of the present invention has high drug compliance due to its rapid antibiotic effect, and has a fast drug penetration rate into the skin or nail and excellent drug permeability. In addition, since the pharmaceutical composition of the present invention does not undergo changes in physical properties (eg, occurrence of precipitation or discoloration, changes in the content of active ingredients, etc.) even when stored at 65°C for 5 weeks or more, the stability of the composition can be ensured.

The present inventors have conducted various studies to further improve the therapeutic effect on Tinea unguium exhibited by the existing efinaconazole single-component preparation, and to develop a pharmaceutical composition excellent in stability without changing in physical properties such as discoloration even during long-term storage.

As a result, the present inventors have accomplished the technique for the pharmaceutical composition of the present invention by revealing that the composition comprising efinaconazole or a pharmaceutically acceptable salt thereof; and octenidine or a pharmaceutically acceptable salt thereof as active ingredients has remarkably elevated antifungal activity and excellent antibacterial activity against Gram-negative bacteria (eg, Pseudomonas aeruginosa) and Gram-positive bacteria (eg, Staphylococcus aureus), exhibits a fast drug penetration rate into the skin or nail, has excellent drug permeability, and exhibits excellent stability even when stored at 65°C for 5 weeks or more.

That is, since the compositions of various Examples of the present invention have a wide range of antimicrobial effects including antifungal and antibacterial effects, it is possible to simultaneously treat the causative microorganisms of Tinea unguium, Tinea corporis and green nail syndrome, and achieve an excellent therapeutic effect. In addition, the composition of the present invention exhibits improved drug permeability to the skin and nail and can maintain thermal stability at high temperature, so it can be used more effectively and usefully as a pharmaceutical composition for therapeutic purposes.

According to various Examples of the present invention, since cyclomethicone is not included in the composition of the present invention, it is possible to solve the problem of harmfulness to the environment due to cyclomethicone.

In addition, according to various Examples of the present invention, industrial safety (eg, safety during storage, handling and/or transportation) can be improved by controlling the content of the vehicle so that the content of the flammable solvent (eg, ethanol) is lowered within the total weight of the composition.

As mentioned above, the present invention has been described in detail through examples, but since the above-described examples have only been described with preferred examples of the present invention, it should not be understood that the present invention is limited only to the examples and the scope of the present invention should be understood by the following claims and their equivalents.

## INDUSTRIAL APPLICABILITY

Since the composition of the present invention has a wide range of antimicrobial effects including antifungal and antibacterial effects, it is possible to simultaneously treat the causative microorganisms of Tinea unguium, Tinea corporis and green nail syndrome, and achieve an excellent therapeutic effect. In addition, the composition of the present invention exhibits improved drug permeability to the skin and nail and can maintain thermal stability at high temperature, so it can be used more effectively and usefully as a pharmaceutical composition for therapeutic purposes.

In addition, according to examples of the present invention, industrial safety (eg, safety during storage, handling and/or transportation) can be improved by controlling the content of the vehicle so that the content of the flammable solvent (eg, ethanol) is lowered within the total weight of the composition.

## Claims

1. A pharmaceutical composition for preventing or treating Tinea, comprising efinaconazole or a pharmaceutically acceptable salt thereof; and octenidine or a pharmaceutically acceptable salt thereof as active ingredients.

2. The pharmaceutical composition for preventing or treating Tinea according to claim 1, wherein the Tinea is Tinea unguium, Tinea corporis or green nail syndrome.

3. The pharmaceutical composition for preventing or treating Tinea according to claim 1, wherein the pharmaceutically acceptable salt of efinaconazole is at least one selected from the group consisting of inorganic ionic salts, inorganic acid salts, organic acid salts, sulfonic acid salts, amino acid salts and amine salts.

4. The pharmaceutical composition for preventing or treating Tinea according to claim 1, wherein the content of efinaconazole or the pharmaceutically acceptable salt thereof is 0.001 ~ 20 weight% based on the total weight of the composition.

5. The pharmaceutical composition for preventing or treating Tinea according to claim 1, wherein the pharmaceutically acceptable salt of octenidine is at least one selected from the group consisting of inorganic ionic salts, inorganic acid salts, organic acid salts, sulfonic acid salts, amino acid salts and amine salts.

6. The pharmaceutical composition for preventing or treating Tinea according to claim 1, wherein the content of octenidine or the pharmaceutically acceptable salt thereof is 0.001 ~ 10 weight% based on the total weight of the composition.

7. The pharmaceutical composition for preventing or treating Tinea according to claim 1, wherein the pharmaceutical composition further includes an additive and the additive is at least one selected from the group consisting of solvents, antioxidants, chelating agents, penetrants, film-formers, disintegrants, binders, lubricants, coating agents, emulsifiers, pressure sensitive adhesives , surfactants, gelling agents, solubilizers, buffering agents, wetting agents, osmotic pressure regulators, aerosol propellants, sweetening agents, coloring agents, flavoring agents, stabilizing agents, bases, pH modifiers and vehicles.

8. The pharmaceutical composition for preventing or treating Tinea according to claim 1, wherein the water content of the pharmaceutical composition measured by Karl Fischer's method is 0.001 ~ 95%.

9. The pharmaceutical composition for preventing or treating Tinea according to claim 1, wherein the pH of the pharmaceutical composition is 2 ~ 10.

10. The pharmaceutical composition for preventing or treating Tinea according to claim 1, wherein the surface tension of the pharmaceutical composition is 1 ~ 75 dyn/cm.

11. The pharmaceutical composition for preventing or treating Tinea according to claim 1, wherein the pharmaceutical composition has a dosage form selected from the group consisting of solutions, ointments, lotions, creams, gels, emulsions, suspensions, powder compacts, pastes, sticks, plasters, patches, tablets, powders, granules, capsules, gums, troches, mucoadhesives, inhalants, microcapsules, detergents, baths, injections, suppositories, liposomes and sprays.

12. The pharmaceutical composition for preventing or treating Tinea according to claim 1, wherein the density of the pharmaceutical composition is 0.001 ~ 2 g/$cm^3$.

13. The pharmaceutical composition for preventing or treating Tinea according to claim 1, wherein the pharmaceutical composition is formulated in the form of inhalation or oral preparations, parenteral preparations including external preparations, and injections.

14. The pharmaceutical composition for preventing or treating Tinea according to claim 1, wherein the daily dosage of the pharmaceutical composition is 0.0001 ~ 300 g/day.

15. The pharmaceutical composition for preventing or treating Tinea according to claim 1, wherein the pharmaceutical composition is colorless or pale yellow, and maintains the same color even after storage at 65°C for at least 5 weeks.

16. A use of the composition according to any one of claims 1 to 15 for the preparation of a medicament for preventing or treating Tinea.

17. A method for preventing or treating Tinea by administering the composition according to any one of claims 1 to 15

to a patient with Tinea.

FIG. 1

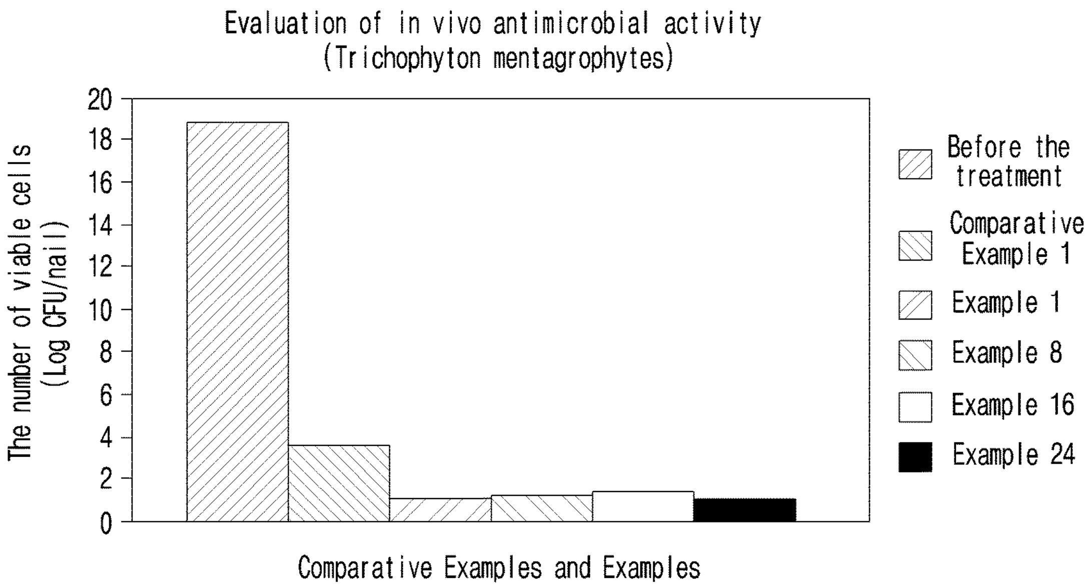
Evaluation of in vivo antimicrobial activity
(Trichophyton mentagrophytes)
The number of viable cells
(Log CFU/nail)
20
18
16
14
12
10
8
6
4
2
0
Before the treatment
Comparative Example 1
Example 1
Example 8
Example 16
Example 24
Comparative Examples and Examples

FIG. 2

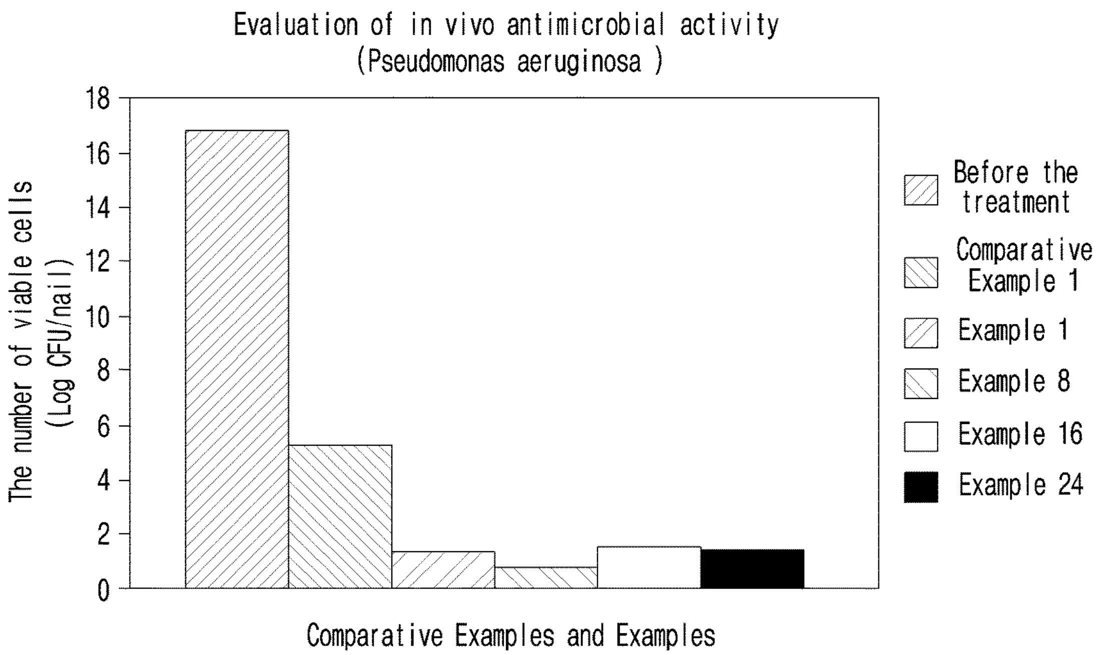
Evaluation of in vivo antimicrobial activity
(Pseudomonas aeruginosa )
The number of viable cells
(Log CFU/nail)
18
16
14
12
10
8
6
4
2
0
Comparative Examples and Examples
Before the treatment
Comparative Example 1
Example 1
Example 8
Example 16
Example 24

FIG. 3

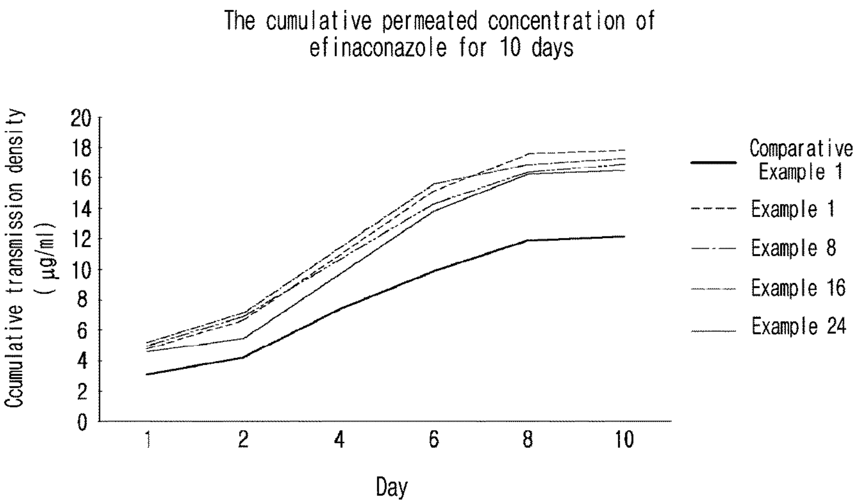
The cumulative permeated concentration of efinaconazole for 10 days
Ccumulative transmission density (µg/ml)
20
18
16
14
12
10
8
6
4
2
0
1
2
4
6
8
10
Day
Comparative Example 1
Example 1
Example 8
Example 16
Example 24

FIG. 4

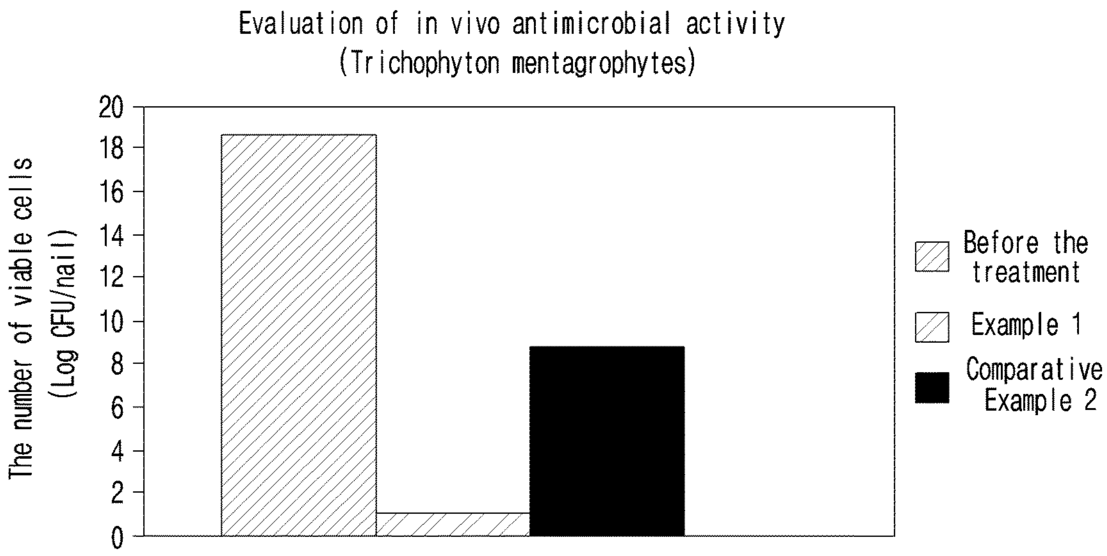
Evaluation of in vivo antimicrobial activity
(Trichophyton mentagrophytes)
The number of viable cells
(Log CFU/nail)
20
18
16
14
12
10
8
6
4
2
0
Before the treatment
Example 1
Comparative Example 2

FIG. 5

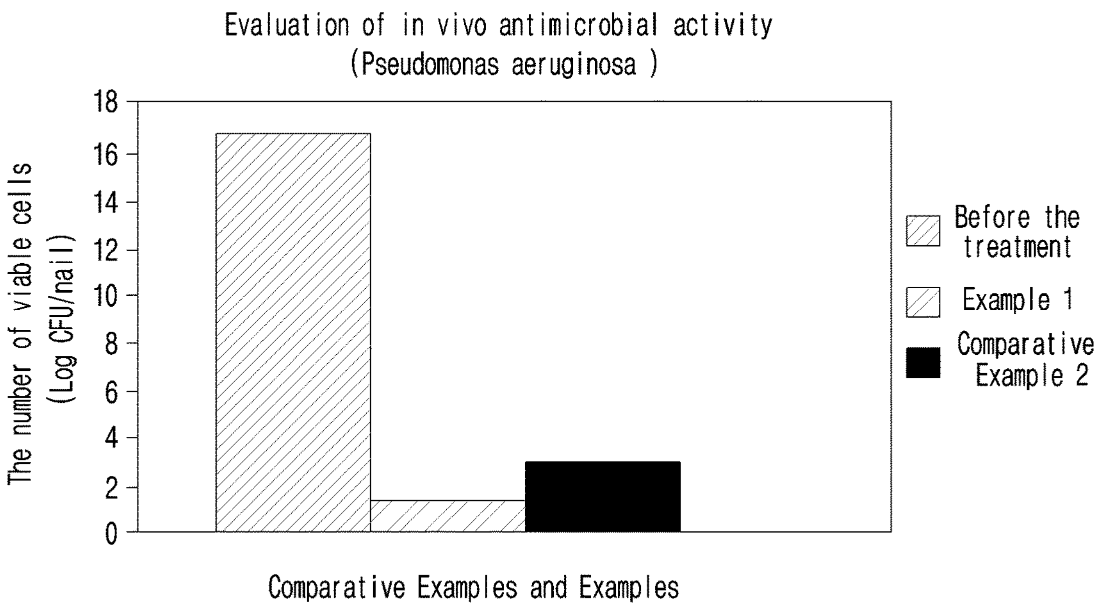
Evaluation of in vivo antimicrobial activity
(Pseudomonas aeruginosa )
The number of viable cells
(Log CFU/nail)
18
16
14
12
10
8
6
4
2
0
Before the treatment
Example 1
Comparative Example 2
Comparative Examples and Examples

FIG. 6

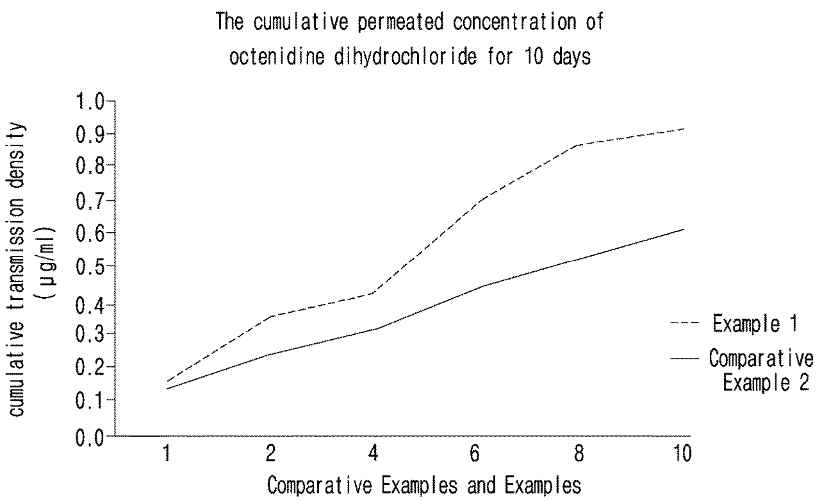
The cumulative permeated concentration of
octenidine dihydrochloride for 10 days
cumulative transmission density
(μg/ml)
1.0
0.9
0.8
0.7
0.6
0.5
0.4
0.3
0.2
0.1
0.0
1
2
4
6
8
10
Comparative Examples and Examples
Example 1
Comparative
Example 2

## INTERNATIONAL SEARCH REPORT

International application No.
**PCT/KR2021/006785**

**A. CLASSIFICATION OF SUBJECT MATTER**

**A61K 31/454**(2006.01)i; **A61K 31/444**(2006.01)i; **A61K 9/00**(2006.01)i; **A61P 31/10**(2006.01)i

According to International Patent Classification (IPC) or to both national classification and IPC

**B. FIELDS SEARCHED**

Minimum documentation searched (classification system followed by classification symbols)

A61K 31/454(2006.01); A01N 47/44(2006.01); A61K 31/05(2006.01); A61K 31/4196(2006.01); A61K 31/444(2006.01); A61K 45/00(2006.01); A61K 47/10(2006.01); A61K 47/14(2006.01); A61K 8/49(2006.01); A61K 9/00(2006.01); A61Q 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 에피나코나졸(Efinaconazole), 옥테니딘(Octenidine), 백선(tinea), 녹농균(Pseudomonas aeruginosa), 항진균(antifungal)

**C. DOCUMENTS CONSIDERED TO BE RELEVANT**

| Category* | Citation of document, with indication, where appropriate, of the relevant passages | Relevant to claim No. |
|---|---|---|
| A | KR 10-2020-0081432 A (KAKEN PHARMACEUTICAL CO., LTD.) 07 July 2020 (2020-07-07) See claims 1-17. | 1-16 |
| A | WO 2015-091692 A1 (ALMIRALL, S.A.) 25 June 2015 (2015-06-25) See paragraph [0150] and claim 1. | 1-16 |
| A | WO 2018-110693 A1 (KANEKA CORPORATION et al.) 21 June 2018 (2018-06-21) See claims 1-25. | 1-16 |
| A | CN 111437276 A (NINGBO INSTITUTE OF TECHNOLOGY, ZHEJIANG UNIVERSITY) 24 July 2020 (2020-07-24) See claims 1-9. | 1-16 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 August 2021** | **30 August 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2021/006785**

**C. DOCUMENTS CONSIDERED TO BE RELEVANT**

| Category* | Citation of document, with indication, where appropriate, of the relevant passages | Relevant to claim No. |
|---|---|---|
| A | ELEWSKI, B. E. et al. Efinaconazole 10% solution in the treatment of toenail onychomycosis: Two phase III multicenter, randomized, double-blind studies. Journal of the American Academy of Dermatology. 2013, vol. 68, no. 4, pp. 600-608.<br>See abstract, pages 603-605 and Figures 3-4. | 1-16 |
| A | KR 10-2016-0087898 A (DOW PHARMACEUTICAL SCIENCES, INC.) 22 July 2016 (2016-07-22)<br>See claims 1-8. | 1-16 |
| A | JP 2011-507978 A (3M INNOVATIVE PROPERTIES CO.) 10 March 2011 (2011-03-10)<br>See entire document. | 1-16 |
| A | KARPINSKI, T. M. Efficacy of octenidine against Pseudomonas aeruginosastrains. European Journal of Biological Research. 2019, vol. 9, no. 3, pp. 135-140.<br>See entire document. | 1-16 |
| PX | KR 10-2217617 B1 (BL&H CO., LTD.) 19 February 2021 (2021-02-19)<br>See claims 1-36.<br>*This document is a published earlier application that serves as a basis for claiming priority of the present international application. | 1-16 |

Form PCT/ISA/210 (second sheet) (July 2019)

**INTERNATIONAL SEARCH REPORT**

International application No.
**PCT/KR2021/006785**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17**
because they relate to subject matter not required to be searched by this Authority, namely:
Claim 17 pertains to a method for treatment of the human body by surgery or therapy, as well as a diagnostic method (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2021/006785**

| Patent document cited in search report | Publication date (day/month/year) | Patent family member(s) | Publication date (day/month/year) |
|---|---|---|---|
| KR 10-2020-0081432 A | 07 July 2020 | AU 2018-358372 A1 | 28 May 2020 |
| | | AU 2018-358372 A1 | 09 May 2019 |
| | | BR 112020008282 A2 | 20 October 2020 |
| | | CA 3081163 A1 | 09 May 2019 |
| | | CN 111295202 A | 16 June 2020 |
| | | EP 3705136 A1 | 09 September 2020 |
| | | IL 274100 D0 | 30 June 2020 |
| | | JP 2020-011967 A | 23 January 2020 |
| | | JP 6582158 B1 | 25 September 2019 |
| | | SG 11202003820 A | 28 May 2020 |
| | | TW 201922247 A | 16 June 2019 |
| | | US 2021-0213002 A1 | 15 July 2021 |
| WO 2015-091692 A1 | 25 June 2015 | AR 098825 A1 | 15 June 2016 |
| | | EP 3082753 A1 | 26 October 2016 |
| | | TW 201605444 A | 16 February 2016 |
| WO 2018-110693 A1 | 21 June 2018 | CN 110087652 A | 02 August 2019 |
| | | EP 3556368 A1 | 23 October 2019 |
| | | EP 3556368 B1 | 26 May 2021 |
| | | US 2020-0078347 A1 | 12 March 2020 |
| | | WO 2018-110693 A1 | 21 June 2018 |
| CN 111437276 A | 24 July 2020 | None | |
| KR 10-2016-0087898 A | 22 July 2016 | CA 2931144 A1 | 28 May 2015 |
| | | CA 3052643 A1 | 28 May 2015 |
| | | CN 105848719 A | 10 August 2016 |
| | | CN 112999223 A | 22 June 2021 |
| | | EP 3071295 A2 | 28 September 2016 |
| | | JP 2017-500374 A | 05 January 2017 |
| | | JP 2020-002153 A | 09 January 2020 |
| | | JP 6611014 B2 | 27 November 2019 |
| | | US 10245257 B2 | 02 April 2019 |
| | | US 10828293 B2 | 10 November 2020 |
| | | US 2015-0148378 A1 | 28 May 2015 |
| | | US 2019-209543 A1 | 11 July 2019 |
| | | US 2021-023069 A1 | 28 January 2021 |
| | | WO 2015-077729 A2 | 28 May 2015 |
| JP 2011-507978 A | 10 March 2011 | AU 2008-347253 A1 | 16 July 2009 |
| | | BR PI0819567 A2 | 05 May 2015 |
| | | CA 2711217 A1 | 16 July 2009 |
| | | CA 2711217 C | 20 June 2017 |
| | | CN 101951779 A | 19 January 2011 |
| | | CN 107019686 A | 08 August 2017 |
| | | EP 2625957 A1 | 14 August 2013 |
| | | US 2010-0282409 A1 | 11 November 2010 |
| | | US 8623935 B2 | 07 January 2014 |
| | | WO 2009-088894 A2 | 16 July 2009 |
| KR 10-2217617 B1 | 19 February 2021 | None | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 102181155
- KR 1020200140681
- KR 1020160068812
- KR 1020180046644


### Non-patent literature cited in the description


- **BYUNG HO OH ; KYU JOONG AHAN.** Drug Therapy of Dermatophytosis. *J Korean Med Assoc,* 2009, vol. 52 (11), 1109-1114
- **UWE WOLLINA ; HOLGER HAENSSLE.** *Dtsch Arztebl Int,* 2016, vol. 113 (29-30), 509-518
- **YANG WON LEE.** Treatment of onychomycosis. *J Korean Med Assoc,* July 2019, vol. 62 (7), 385-391
- **SHITAL AMIN, POOJARY.** Topical Antifungals; A Review and their Role Management of Dermatophytoses. *Clinical Dermatology Review,* 2017, vol. 1, s24-s29
- *CHEMICAL ABSTRACTS,* 164650-44-6
- *CHEMICAL ABSTRACTS,* 71251-02-0
- *CHEMICAL ABSTRACTS,* 70775-75-6
- **RAYMOND C. ROWE.** Handbook of Pharmaceutical Excipients